# EUROPEAN PATENT APPLICATION

(11) **EP 4 726 733 A1**
(43) Date of publication of application: **15.04.2026**
(21) Application number: 24921769.6
(22) Date of filing: 18.12.2024
(51) Int. Cl.: G16H 40/20

(54) **HEALTH MANAGEMENT METHOD AND RELATED DEVICE**

(30) Priority: 29.01.2024 CN 202410120030
(71) Applicant: Huawei Technologies Co., Ltd., Shenzhen, Guangdong 518129 (CN)
(72) Inventor: WEI, Kangming, Shenzhen, Guangdong 518129 (CN); JIN, Yufeng, Shenzhen, Guangdong 518129 (CN); DONG, Yufei, Shenzhen, Guangdong 518129 (CN)
(74) Representative: Goddar, Heinz J.
(86) International application number: PCT/CN2024/140321
(87) International publication number: WO 2025/161747

(57) **Abstract**

A health management method and a related device are provided, to learn about daily behavior and a physical status of a family member in real time. For example, a first device receives a notification message, where the notification message is used to notify a first user of a status of a second user. The first user is a user corresponding to the first device. The second user has an association relationship with the first user, and the status of the second user includes daily behavior and/or a physical status of the second user. The first device displays the notification message. In this manner, the first user can learn about the status of the second user, so that interaction is improved, an accident of the second user (for example, an elderly person) can be further avoided, and experience is good.

## Description

### CROSS-REFERENCE TO RELATED APPLICATIONS

This application claims priority to Chinese Patent Application No. 202410120030.X, field with the China National Intellectual Property Administration on January 29, 2024 and entitled "HEALTH MANAGEMENT METHOD AND RELATED DEVICE", which is incorporated herein by reference in its entirety.

### TECHNICAL FIELD

This application relates to the field of terminal technologies, and in particular, to a health management method and a related device.

### BACKGROUND

Vulnerable groups such as elderly people and disabled people are prone to accidents due to physical reasons. Therefore, real-time attention needs to be paid to daily life of these groups to prevent accidents. How to learn about daily behavior and physical statuses of these groups in time is an issue of concern to current society.

### SUMMARY

Embodiments of this application provide a health management method and a related device, to learn about daily behavior and a physical status of a family member in real time, and improve user experience.

According to a first aspect, a health management method is provided, and is applicable to a first device. The method includes: receiving a notification message, where the notification message is used to notify a first user of a status of a second user, the first user is a user corresponding to the first device, the second user has an association relationship with the first user, and the status of the second user includes daily behavior and/or a physical status of the second user; and displaying the notification message.

In this embodiment of this application, the first user (for example, a daughter) may be notified of the status of the second user (for example, an elderly person), so that interaction can be improved, an accident of the second user (for example, the elderly person) can be further avoided, and experience is good.

In a possible design, the status of the second user includes at least one of the following: the second user executes a first preset event; the second user does not execute a second preset event; and the second user triggers a specific function of a second device, where the second device is a device of the second user.

In this embodiment of this application, when the second user executes an event, does not execute an event, or triggers a specific function, the first user may be notified, so that the first user learns about the status of the second user in time, and security of the second user is ensured.

In a possible design, that the second user executes the first preset event includes: the second user uses a physical examination function of the second device to perform a physical examination, where the second device is a device corresponding to the second user.

In this embodiment of this application, when the second user uses the physical examination function (for example, a micro-physical examination function), the first user may be notified, so that the first user can view a physical examination report of the second user quickly to a greatest extent, and learn about the physical status of the second user in time.

In a possible design, that the second user does not execute the second preset event includes: the second user does not use, for more than first duration, a physical examination function of the second device to perform a physical examination, where the second device is a device corresponding to the second user; or the second user does not exercise or has an amount of exercise less than a first threshold for more than second duration.

In this embodiment of this application, when the second user does not perform the physical examination for a long time or does not exercise for a long time, the first user may be notified, to implement supervision on the second user.

In a possible design, that the second user triggers the specific function of the second device includes: the second user triggers an SOS button of the second device; or the second user triggers a fall detection function of the second device and it is detected that the second user falls.

In this embodiment of this application, when the second user triggers SOS or falls, the first user may be notified in time, so that the first user learns about a situation of the second user in time, to avoid a danger to a greatest extent.

In a possible design, the status of the second user includes at least one of the following: the second user moves out of a first geo-fence and/or moves into a second geo-fence; and a physical examination result of the second user is abnormal.

In this embodiment of this application, when the second user moves out of the first geo-fence (a home, a housing estate, or the like) or enters the second geo-fence (a street, a seaside, or the like), the first user may be notified in time, to avoid a danger to a greatest extent.

In a possible design, the notification message includes a first shortcut, and the method further includes: receiving an operation on the first shortcut, and displaying the status of the second user.

In this embodiment of this application, the notification message received by the first device includes the first shortcut. In this way, the first user can quickly view the status of the second user, and can view the status of the second user without a complex operation process, which improves operation convenience. Optionally, the first shortcut may be a button.

In a possible design, the notification message includes a second shortcut, and the method further includes: receiving an operation on the second shortcut, and sending a first message to the second device, where the second device is a device of the second user, and the first message is used to comment on the status of the second user.

In this embodiment of this application, the notification message received by the first device includes the second shortcut. In this way, the first user can quickly comment on the status of the second user, and can comment on the status of the second user without a complex operation process, which improves operation convenience.

In a possible design, the status of the second user includes that the second user does not execute the second preset event, the notification message includes a third shortcut, and the method further includes: receiving an operation on the third shortcut, and contacting the second user or sending a second message to the second device, where the second device is a device of the second user, and the second message is used to prompt the second user to execute the second preset event.

In this embodiment of this application, the notification message received by the first device includes the third shortcut. In this way, the first user can quickly remind the second user to execute an event, which improves operation convenience.

In a possible design, the method further includes: when an operation used to cancel display of the notification message is received, making no response or outputting prompt information, where the prompt information indicates that display of the notification message is unable to be canceled; or when an operation used to contact the second user is received, canceling display of the notification message.

In this embodiment of this application, the notification message on the first device may dominate a screen for display. For example, when the first user does not contact the second user, display of the notification message cannot be canceled. Display of the notification message may be canceled only when the first user contacts the second user. In this way, the first user can be forced to contact the second user, so that a case in which the second user is in danger and no one rescues the second user in time because the first user ignores the notification message is avoided.

In a possible design, before receiving the notification message, the method further includes: sending a first request to the second device, where the first request is used to request to establish the association relationship between the first user and the second user, and the second device is a device of the second user; and receiving a consent instruction of the second user.

In this embodiment of this application, the association relationship (for example, a friendship) may be established between the first user and the second user. In this way, the first user may be notified of the status of the second user, so that interaction can be improved, an accident of the second user (for example, the elderly person) can be further avoided, and experience is good.

In a possible design, the method further includes: sending a second request to the second device, where the second request is used to request to obtain access permission for the status of the second user, and the second device is a device of the second user; and receiving a consent instruction of the second user.

In this embodiment of this application, if the first user wants to access the status of the second user, the first user may request access. The first user may access the status of the second user with consent of the second user. In this way, a case in which all people can access the status of the second user can be avoided, and privacy is improved.

In a possible design, the status of the second user includes the physical status of the second user, and the method further includes: obtaining a diagnosis result based on the physical status of the second user; and sending the diagnosis result to the second device, where the second device is a device of the second user.

In this embodiment of this application, the first user may help the second user with consultation, and send a consultation result to the second user, to resolve a problem of inconvenient consultation of the second user (for example, the elderly person).

According to a second aspect, a health management method is further provided, and is applicable to a second device. The second device may be, for example, a mobile phone. The method includes: determining a status of a second user, where the second user is a user corresponding to the second device, and the status of the second user includes daily behavior and/or a physical status of the second user; and sending a notification message to a first device, where the notification message is used to notify a first user of the status of the second user, the first user has an association relationship with the second user, and the first device is a device of the first user.

In a possible design, the status of the second user includes at least one of the following: the second user executes a first preset event; the second user does not execute a second preset event; and the second user triggers a specific function of a third device, where the third device is a device of the second user.

In a possible design, that the second user executes the first preset event includes: the second user uses a physical examination function of the third device to perform a physical examination, where the third device is a device corresponding to the second user.

In a possible design, that the second user does not execute the second preset event includes: the second user does not use, for more than first duration, a physical examination function of the third device to perform a physical examination, where the third device is a device corresponding to the second user; or the second user does not exercise or has an amount of exercise less than a first threshold for more than second duration.

In a possible design, that the second user triggers the specific function of the third device includes: the second user triggers an SOS button of the third device; or the second user triggers a fall detection function of the third device and it is detected that the second user falls.

In a possible design, the status of the second user includes at least one of the following: the second user moves out of a first geo-fence and/or moves into a second geo-fence; and a physical examination result of the second user is abnormal.

In a possible design, the method further includes: receiving a first message sent by the first device, where the first message is used to comment on the status of the second user.

In a possible design, the method further includes: receiving an operation on a first shortcut, and viewing or replying to a comment on the second user.

In this embodiment of this application, the first user may be notified of the status of the second user, and the first device of the first user may send the first message to the second device of the second user to comment on the status of the second user. The second device may display the first shortcut based on the first message. In this way, the second user can quickly view comment content or quickly reply to the comment, which improves operation convenience.

In a possible design, the status of the second user includes that the second user does not execute the second preset event, and the method further includes: receiving a second message sent by the first device, where the second message is used to prompt the second user to execute the second preset event.

In a possible design, the method further includes: receiving an operation on a second shortcut, and executing the second preset event.

In this embodiment of this application, when the second user does not execute an event for a long time, the first user may be notified. The first device of the first user may send the second message to the second device of the second user to remind the second user. The second device may display the second shortcut based on the second message. In this way, the second user can quickly execute the event, which improves operation convenience.

In a possible design, the method further includes: displaying the second message; and when an operation used to cancel display of the second message is received, making no response or outputting prompt information, where the prompt information indicates that display of the second message is unable to be canceled; or after it is determined that the second user executes the second preset event, canceling display of the second message.

In this embodiment of this application, the second message may dominate a screen for display. For example, when the second user does not execute the event, display of the second message cannot be canceled. Display of the second message can be canceled only when the second user executes the event. In this way, the second user can be forced to execute the event.

In a possible design, the method further includes: displaying first prompt information, where the first prompt information indicates that the first user has viewed the status of the second user.

In this embodiment of this application, the first user may be notified of the status of the second user. After the first user views the status of the second user, the second device of the second user may indicate that the first user has viewed the status of the second user.

In a possible design, before sending the notification message to the first device, the method further includes: receiving a first request sent by the first device, where the first request is used to request to establish the association relationship between the first user and the second user; and receiving a first operation, where the first operation indicates consent to establishing the association relationship between the first user and the second user.

In a possible design, the method further includes: receiving a second request sent by the first device, where the second request is used to request to obtain access permission for the status of the second user; and receiving a second operation, where the first operation indicates consent to granting the access permission.

In a possible design, the status of the second user includes the physical status of the second user, and the method further includes: receiving a diagnosis result sent by the first device, where the diagnosis result is obtained based on the physical status of the second user; and displaying the diagnosis result.

According to a third aspect, a communication system is further provided, including a first device and a second device.

The first device is configured to perform the method steps according to the first aspect.

The second device is configured to perform the method steps according to the second aspect.

According to a fourth aspect, an electronic device is further provided, including:
a processor, a memory, and one or more programs.

The one or more programs are stored in the memory. The one or more programs include instructions. When the instructions are executed by the processor, the electronic device is enabled to perform the method according to the first aspect or the second aspect.

According to a fifth aspect, a computer-readable storage medium is further provided. The computer-readable storage medium is configured to store a computer program. When the computer program is run on a computer, the computer is enabled to perform the method according to the first aspect or the second aspect.

According to a sixth aspect, a computer program product is further provided, including a computer program. When the computer program is run on a computer, the computer is enabled to perform the method according to the first aspect or the second aspect.

According to a seventh aspect, a chip is further provided. The chip is coupled to a memory in an electronic device, and is configured to: invoke a computer program stored in the memory, and perform the technical solutions according to the first aspect or the second aspect of embodiments of this application. In embodiments of this application, "coupling" means that two components are directly or indirectly combined with each other.

For technical effects that can be achieved by the second aspect to the seventh aspect, refer to descriptions of the technical effects that can be achieved by corresponding design solutions in the first aspect. Details are not described herein again in this application.

### BRIEF DESCRIPTION OF DRAWINGS

FIG. 1 is a diagram of a structure of a communication system according to an embodiment of this application;
FIG. 2 is a schematic flowchart of a health management method according to an embodiment of this application;
FIG. 3A(a) to FIG. 3B(c) are diagrams in which a user A adds a user B as a friend according to an embodiment of this application;
FIG. 3C(a) to FIG. 3E(b) are diagrams in which a user A applies to a user B for data access permission according to an embodiment of this application;
FIG. 4 is another schematic flowchart of a health management method according to an embodiment of this application;
FIG. 5A(a) to FIG. 5B(b) are diagrams in which a user A is notified of a status of a user B according to an embodiment of this application;
FIG. 5C(a) to FIG. 5D(b) are diagrams in which a user B receives a reminder or a comment from a user A according to an embodiment of this application;
FIG. 6(a) to FIG. 6(c) are another diagram in which a user A is notified of a status of a user B according to an embodiment of this application;
FIG. 7 is still another schematic flowchart of a health management method according to an embodiment of this application;
FIG. 8A(a) to FIG. 8B(b) are diagrams in which a user A helps a user B with online consultation according to an embodiment of this application;
FIG. 9 is a diagram of an electronic device according to an embodiment of this application; and
FIG. 10 is another diagram of an electronic device according to an embodiment of this application.

### DESCRIPTION OF EMBODIMENTS

The following describes some terms in embodiments of this application, to facilitate understanding of a person skilled in the art.
(1) In embodiments of this application, "at least one" includes one or more, and "a plurality of" means two or more. In addition, it should be understood that in descriptions of this specification, words such as "first" and "second" are merely intended for purposes of description, and should not be understood as expressing or implying relative importance or a sequence. For example, a first device and a second device do not represent an importance degree of the first device and the second device or represent a sequence of the first device and the second device, and are merely used for distinguishing and description. A term "and/or" in embodiments of this application describes only an association relationship and represents that three relationships may exist. For example, A and/or B may represent the following three cases: Only A exists, both A and B exist, and only B exists. In addition, the character "/" in this specification generally indicates an "or" relationship between the associated objects.
(2) The orientation terms mentioned in embodiments of this application, for example, "up", "down", "left", "right", "inside", and "outside", are merely directions based on the accompanying drawings. Therefore, the orientation terms are used to better and more clearly describe and understand embodiments of this application, instead of indicating or implying that a specified apparatus or element should have a specific orientation and be constructed and operated in a specific orientation. Therefore, this cannot be understood as a limitation on embodiments of this application.
(3) Reference to "an embodiment", "in some examples", "some embodiments", or the like described in embodiments of this application means that one or more embodiments of this specification include a specific feature, structure, or characteristic described with reference to the embodiment. Therefore, statements such as "in some examples", "in an embodiment", "in some embodiments", "in some other embodiments", and "in other embodiments" that appear at different places in this specification do not necessarily mean reference to a same embodiment, instead, the statements mean "one or more but not all of embodiments", unless otherwise specifically emphasized. The terms "include", "comprise", "have", and their variants all mean "include but are not limited to", unless otherwise specifically emphasized in another manner.
(4) The "status of the user" in embodiments of this application may include daily behavior and/or a physical status of the user. For example, for the status of the user, refer to Table 1.

**Table 1: Status of a user**

| | | |
|---|---|---|
| Status of the user | Daily behavior of the user | Exercise |
| | | Diet |
| | | Sleeping/Waking up |
| | | Taking medicine |
| | | ··· |
| | Physical status of the user | Heart rate |
| | | Blood pressure |
| | | Blood oxygen |
| | | Body fat percentage |
| | | Micro-physical examination |

As mentioned above, how to learn about daily behavior and physical statuses of vulnerable groups such as elderly people and disabled people in time is an issue of concern currently. Therefore, embodiments of this application provide a health management method. According to the method, daily behavior and/or a physical status of a family member can be learned about in time, and the daily behavior and/or the physical status of the family member can be further managed to prevent an accident. Optionally, as shown in Table 1, the daily behavior of the family member includes work and rest, diet, exercise, or the like of the family member, and the physical status of the family member includes a heart rate, blood pressure, blood oxygen, or the like of the family member.

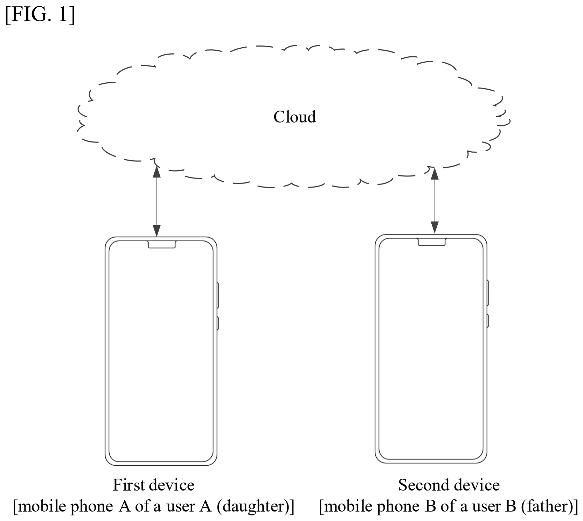

The health management method provided in embodiments of this application is applicable to a communication system. For example, FIG. 1 is a diagram of a communication system according to an embodiment of this application. As shown in FIG. 1, a communication system includes a first device, a second device, and a cloud. In FIG. 1, for example, the first device is a mobile phone of a user A, and the second device is a mobile phone of a user B. In this embodiment of this application, the user A may view daily behavior and/or a physical status of the user B by using the mobile phone A. For example, the user A is a daughter, and the user B is a father. The daughter may view daily behavior and/or a physical status of the father by using the mobile phone A. Therefore, the daughter may learn about the daily behavior and/or the physical status of the father in real time to avoid an accident of the elderly person. Certainly, the father may also view daily behavior and/or a physical status of the daughter by using the mobile phone B.

It should be noted that, in FIG. 1, it is considered that a distance between the mobile phone A and the mobile phone B may be long. Therefore, the mobile phone A may establish long-distance communication with the mobile phone B by using a cloud, to view the daily behavior and/or the physical status of the father on the mobile phone B. It may be understood that there is another scenario. For example, the distance between the mobile phone A and the mobile phone B is short. For example, both the daughter and the father are at home. In this case, short-distance communication may be established between the mobile phone A and the mobile phone B. The daily behavior and/or the physical status of the father on the mobile phone B is viewed by using the mobile phone A in a short-distance communication manner. Optionally, the short-distance communication includes, for example, Bluetooth (Bluetooth), Bluetooth low energy (Bluetooth Low Energy, BLE), near field communication (Near Field Communication, NFC), and ultra-wideband (Ultra-wideband, UWB). This is not limited in embodiments of this application. Alternatively, the mobile phone A may establish communication with the mobile phone B by using a router at home, a smart home device at home, or the like. That is, the daily behavior and/or the physical status of the father on the mobile phone B is viewed by using the router or the smart home device at home. Therefore, in this case, the cloud in FIG. 1 may be replaced with the router or the smart home device at home. In conclusion, the cloud in FIG. 1 may exist or may not exist. Therefore, the cloud in FIG. 1 is represented by using a dashed line. The following mainly uses an example in which FIG. 1 includes the cloud for description.

It should be noted that, in FIG. 1, for example, the first device is the mobile phone A. It may be understood that the first device may alternatively be another device other than a mobile phone, for example, may alternatively be a personal computer (Personal Computer, PC), where the PC may include a device such as a tablet computer, a notebook computer, or a desktop computer; or may be a wearable device such as a watch or a band; or may be a smart home device such as a television or a refrigerator, or may be a vehicle-mounted device; or may be a virtual reality (Virtual Reality, VR) device, an augmented reality (Augmented Reality, AR) device, a mixed reality (Mixed Reality, MR) device, or the like. In conclusion, a specific type of the first device is not limited in embodiments of this application. Similarly, in FIG. 1, for example, the second device is the mobile phone B. It may be understood that the second device may alternatively be another device, for example, may alternatively be a PC, where the PC may include a device such as a tablet computer, a notebook computer, or a desktop computer; or may be a wearable device such as a watch or a band; or may be a smart home device such as a television or a refrigerator; or may be a vehicle-mounted device; or may be a VR device, an AR device, an MR device, or the like. In conclusion, a specific type of the second device is not limited in embodiments of this application. Optionally, the first device and the second device may be devices of a same type or devices of different types. This is not limited in embodiments of this application.

The following continues to use the communication system in FIG. 1 as an example, and uses an example in which the user A is a daughter and the user B is a father in FIG. 1 to describe the technical solutions provided in embodiments of this application.

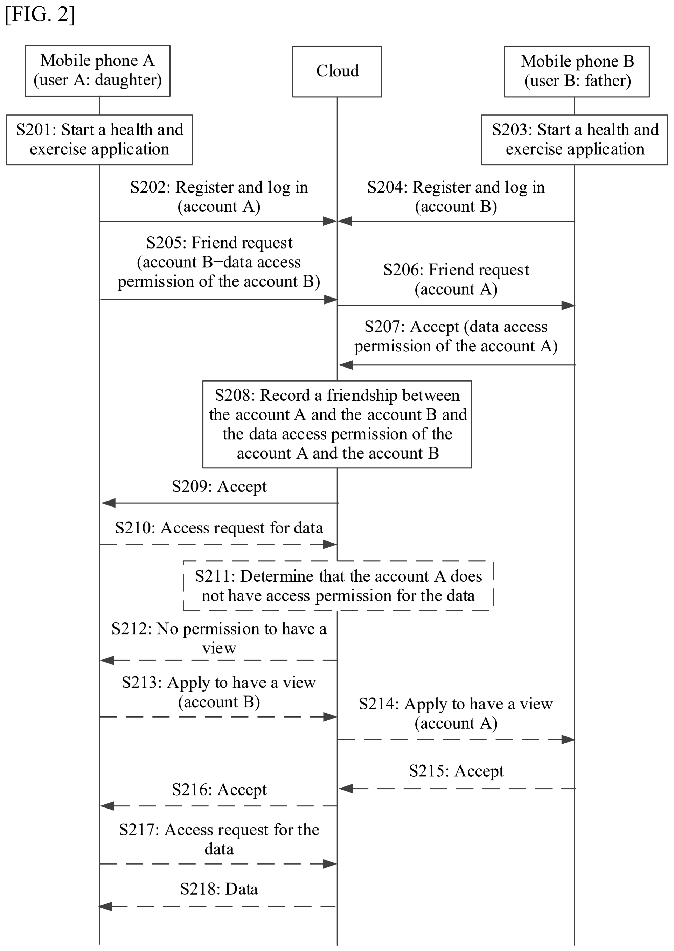

FIG. 2 is a schematic flowchart of a health management method according to an embodiment of this application. The method may be applied to the communication system shown in FIG. 1. As shown in FIG. 2, the method includes the following steps.

S201: A mobile phone A starts a health and exercise application.

S202: The mobile phone A registers with and logs in to a cloud by using an account A.

S203: A mobile phone B starts a health cloud application.

S204: The mobile phone B registers with and logs in to the cloud by using an account B.

For example, the health and exercise application may be a Huawei Health application. Optionally, the health and exercise application may be a system application or a third-party application. Optionally, the health and exercise application may alternatively be replaced with another application. This is not limited in embodiments of this application. In some examples, the account A and/or the account B may be a system account, for example, a Huawei system account. A process in which the mobile phone A and the mobile phone B register with and log in to the cloud by using the health and exercise application is not described in this embodiment of this application. In conclusion, the health and exercise application on the mobile phone A is logged in to in the cloud by using the account A, and the health and exercise application on the mobile phone B is logged in to in the cloud by using the account B.

In this embodiment of this application, the user A (daughter) may add the user B (father) as a friend by using the health and exercise application on the mobile phone A, and then view daily behavior and/or a physical status of the user B. A friend adding process may include S205 to S209 in the following.

S205: The mobile phone A sends a friend request to the cloud, to request to add the user B as a friend. The friend request includes the account B of the user B.

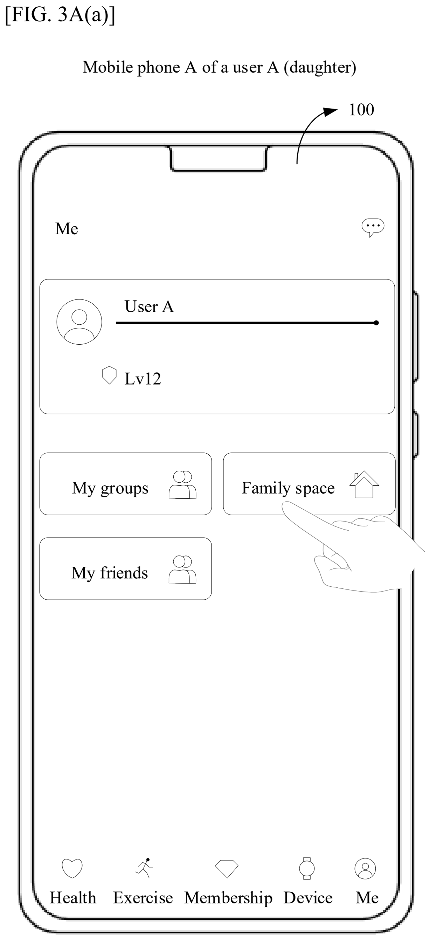

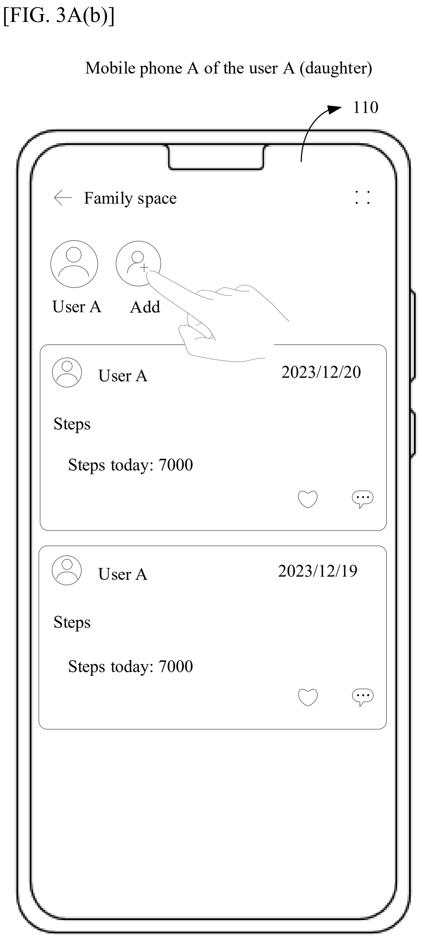

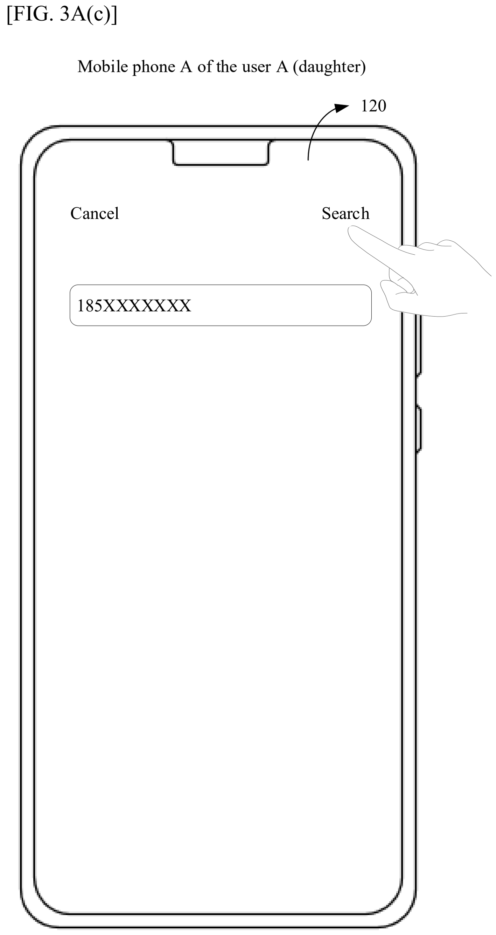

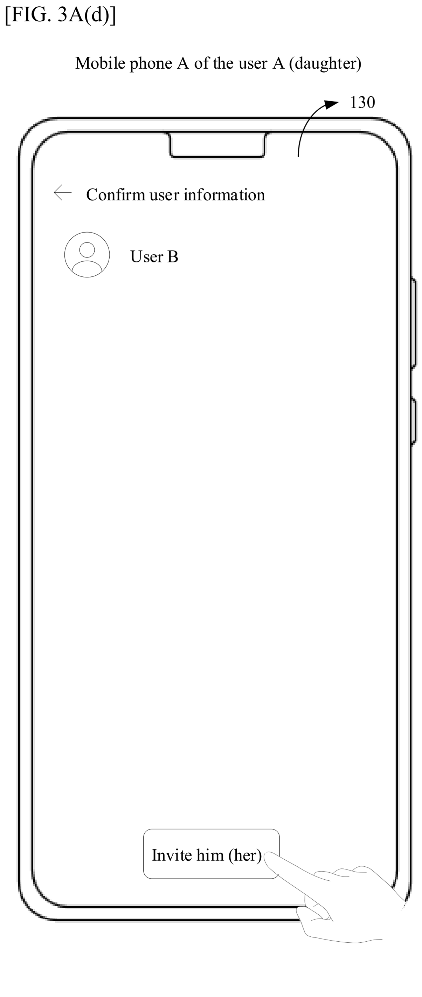

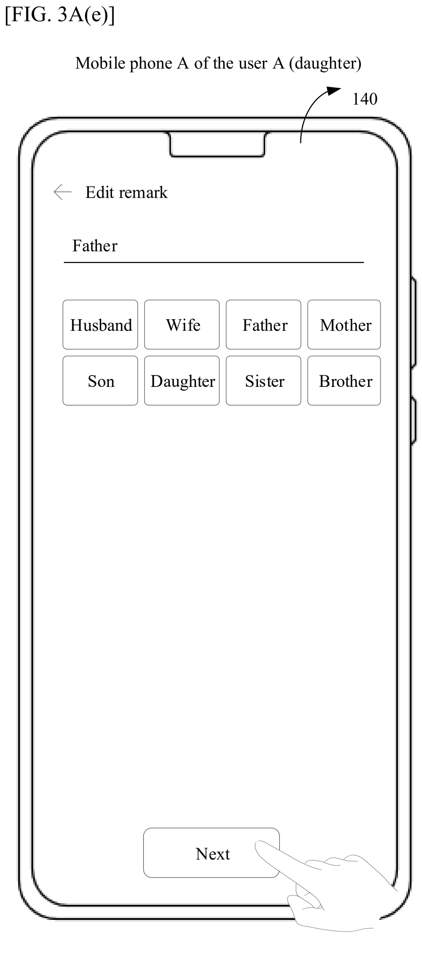

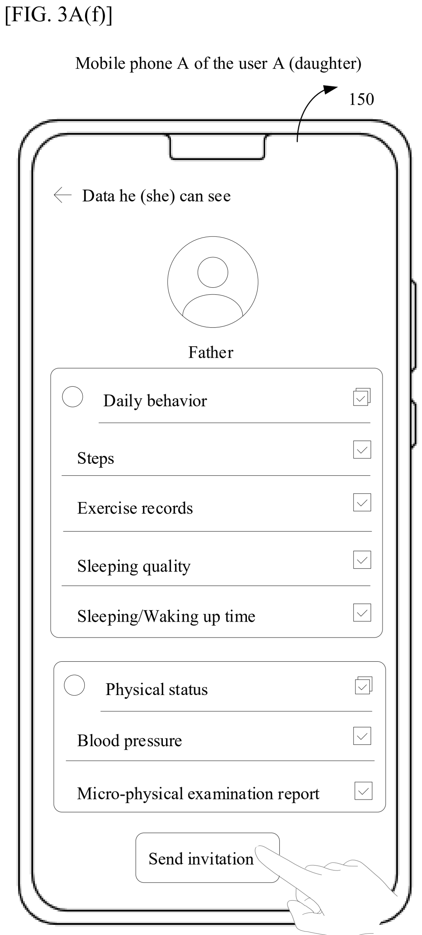

For example, as shown in FIG. 3A(a), the mobile phone A displays an interface 100. The interface 100 may be an interface of the health and exercise application on the mobile phone A. The interface 100 includes a "Family space" button. When receiving an operation on the button, the mobile phone A displays an interface 110 shown in FIG. 3A(b). The interface 110 includes an "Add" button. When receiving an operation on the "Add" button, the mobile phone A displays an interface 120 shown in FIG. 3A(c). The interface 120 includes an input box. The user A may enter the account B of the user B, for example, a mobile phone number of the user B, in the input box. When receiving an operation on a "Search" button on the interface 120, the mobile phone A displays an interface 130 shown in FIG. 3A(d). The interface 130 includes a search result, for example, a profile photo and a nickname of the user B (father). When receiving a button for "Invite him (her)", the mobile phone A displays an interface 140 shown in FIG. 3A(e). The interface 140 is used for setting a remark for the user B, for example, setting the remark for the user B as "Father". It should be noted that the foregoing uses an example in which the user B is added as a friend by using "Family space" in FIG. 3A(a) as an entry. During actual application, the user B may alternatively be added as a friend by using another button, for example, by using a "My friend" button in FIG. 3A(a). In some examples, a friend added through "Family space" is considered to have a family relationship with the user A, and a friend added through "My friend" is considered to have a friendship with the user A.

In some examples, in FIG. 3A(e), when receiving an operation on a "Next" button, the mobile phone A sends the friend request to the cloud. The friend request may include the account B of the user B. Optionally, the friend request may further include the account A of the user A. Optionally, the friend request may further include the remark for the user B, for example, "Father". Optionally, the friend request may further include a relationship between the user B and the user A. A manner of determining the relationship is: Because the user B is added as a friend through "Family space", it is determined that the relationship between the user B and the user A is the family relationship. Alternatively, another manner of determining the relationship is: Because the remark for the user B is "Father", it is determined that the relationship between the user B and the user A is the family relationship. Certainly, the relationship may alternatively be determined in another manner. This is not limited in embodiments of this application.

In some other examples, in FIG. 3A(e), when receiving the operation on the "Next" button, the mobile phone A displays an interface 150 shown in FIG. 3A(f). The interface 150 is used for setting data to be disclosed to the user B (father). For example, the interface 150 mainly includes two pieces of data. One is the daily behavior, and the other is the physical status. The two pieces of data may each include one or more pieces of data. The user A (daughter) may choose a piece of data to be disclosed to the user B (father). For example, in FIG. 3A(f), the user A selects all the data. In other words, all the data is disclosed to the user B. In this case, when receiving an operation on a "Send invitation" button, the mobile phone A sends the friend request to the cloud. The friend request includes the account B of the user B. Optionally, the friend request may further include the account A of the user A. The friend request may further include data access permission of the user B, to indicate the data to be disclosed to the user B (father). It should be noted that during actual application, the interface 150 may include more or fewer data options than those shown in FIG. 3A(f), and there may be another layout manner between the data options on the interface 150. This is not limited in embodiments of this application. It should be noted that, in FIG. 3A(f), the user A sets the data to be disclosed to the user B. Optionally, the data may be permanently disclosed or temporarily disclosed. The temporary disclosure is used as an example. For example, the data is disclosed to the user B for preset duration, and disclosure of the data to the user B is canceled after the preset duration. Optionally, the preset duration may be three days, five days, one week, or the like. This is not limited in embodiments of this application. The preset duration may be set by a system by default, or may be specified by the user. This is not limited in embodiments of this application.

S206: The cloud sends the friend request to the mobile phone B, where the friend request includes the account A of the user A.

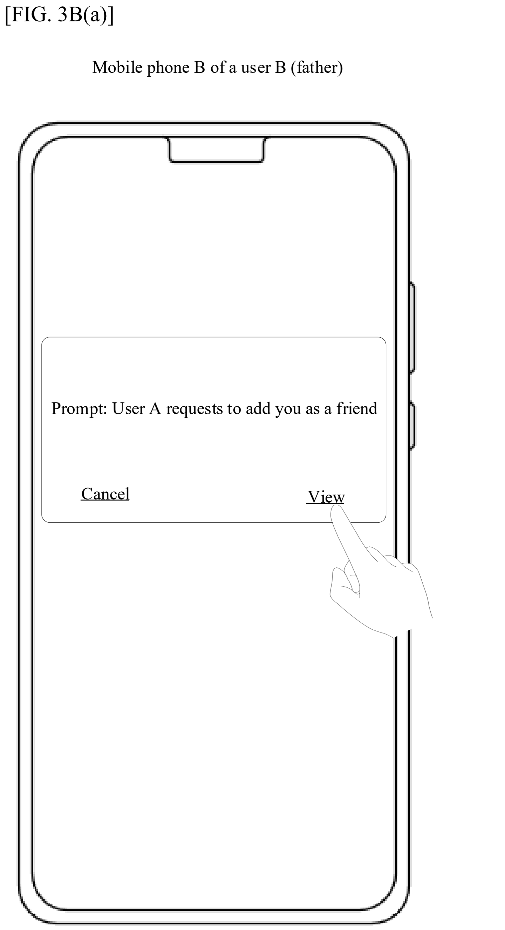

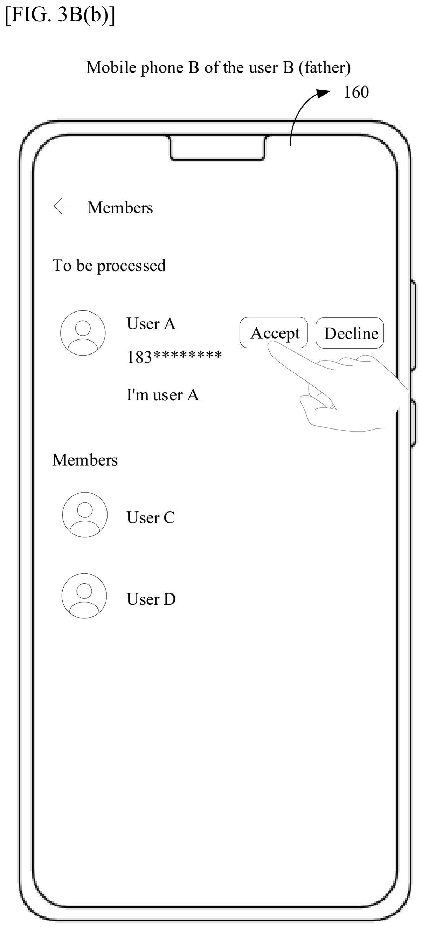

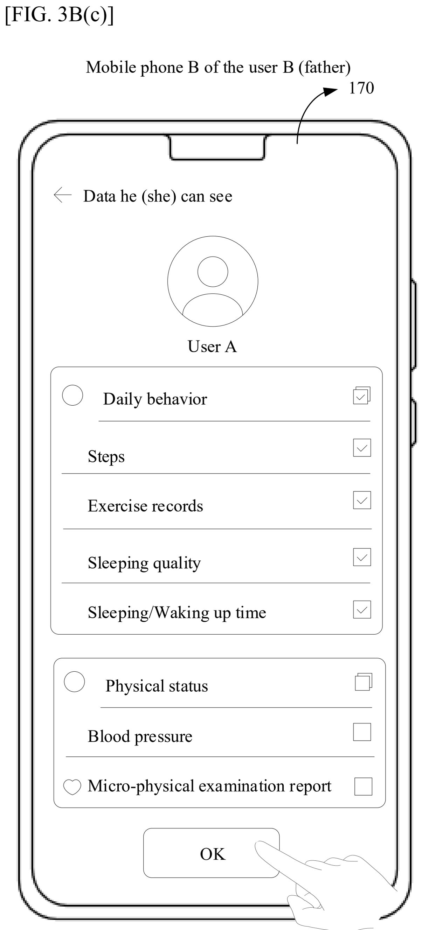

In some examples, when receiving the friend request sent by the cloud, the mobile phone B may output prompt information, to indicate that the user A requests to add a friend. For example, refer to FIG. 3B(a). The mobile phone B displays prompt information: User A requests to add you as a friend. Optionally, "User A" in the prompt information in FIG. 3B(a) may be a nickname of the user A, and the nickname may be a network name set by the user A. Alternatively, "User A" in the prompt information may be replaced with "Daughter". That is, the prompt information is: Daughter requests to add you as a friend. In this case, the mobile phone B needs to first determine the relationship (for example, a father-daughter relationship) between the user A and the user B. In a possible manner, the friend request received by the mobile phone B includes an account of the mobile phone A, for example, a mobile phone number of the mobile phone A. The mobile phone B queries an address book, determines that a remark for the mobile phone number is the daughter, and determines that the user A is the daughter. In another possible manner, after receiving the friend request of the mobile phone A, the cloud may infer that the user A is the daughter of the user B based on information (gender, age, and the like) of the user A and the remark "Father" that the user A sets for the user B. Therefore, the friend request sent by the cloud to the mobile phone B may indicate that the user A is the daughter of the user B. Certainly, the relationship between the user A and the user B may alternatively be determined in another manner. Examples are not enumerated one by one in this embodiment of this application.

S207: The mobile phone B sends a consent instruction to the cloud, to indicate that the friend request of the user A is accepted.

Still refer to FIG. 3B(a). When receiving an operation on a "View" button, the mobile phone displays an interface 160 shown in FIG. 3B(b). The interface 160 may be an interface of the health and exercise application on the mobile phone B. The interface 160 includes a to-be-processed message, for example, the friend request of the user A. The mobile phone B further displays an "Accept" button and a "Decline" button.

In some examples, when receiving an operation on the "Accept" button, the mobile phone B sends the consent instruction to the cloud.

In some other examples, when receiving the operation on the "Accept" button, the mobile phone B may display an interface 170 shown in FIG. 3B(c). The interface 170 is used for setting data to be disclosed to the user A (daughter). For example, the user B selects an option of daily behavior, but does not select an option of the physical status. In this case, the daily behavior is disclosed to the user A, and the physical status is not disclosed to the user A. When receiving an operation on an "OK" button, the mobile phone B sends a consent instruction to the cloud. In this case, the consent instruction may include data access permission of the user A, to indicate data to be disclosed to the user A (daughter). It should be noted that, in FIG. 3B(c), the user B sets the data to be disclosed to the user A. Optionally, the data may be permanently disclosed or temporarily disclosed. This is not limited in embodiments of this application.

S208: The cloud records a friendship between the account A and the account B and data access permission of the account A and the account B.

For example, in FIG. 3A(f), the data disclosed by the daughter to the father includes the daily behavior and the physical status of the daughter. Therefore, the data access permission of the user B (father) includes the daily behavior and the physical status of the daughter. For example, in FIG. 3B(c), the data disclosed by the father to the daughter includes the daily behavior of the father, but does not include the physical status of the father. Therefore, the data access permission of the user A (daughter) includes the daily behavior of the father, but does not include the physical status of the father.

S209: The cloud sends the consent instruction to the mobile phone A, to indicate that the friend request is accepted.

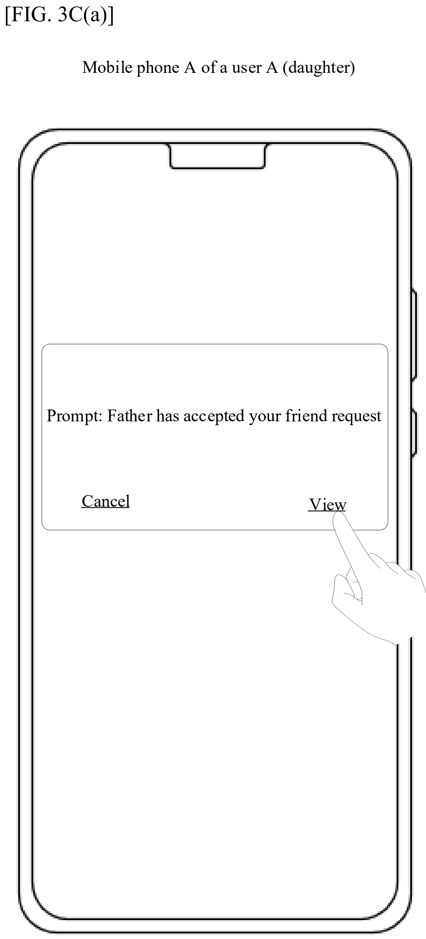

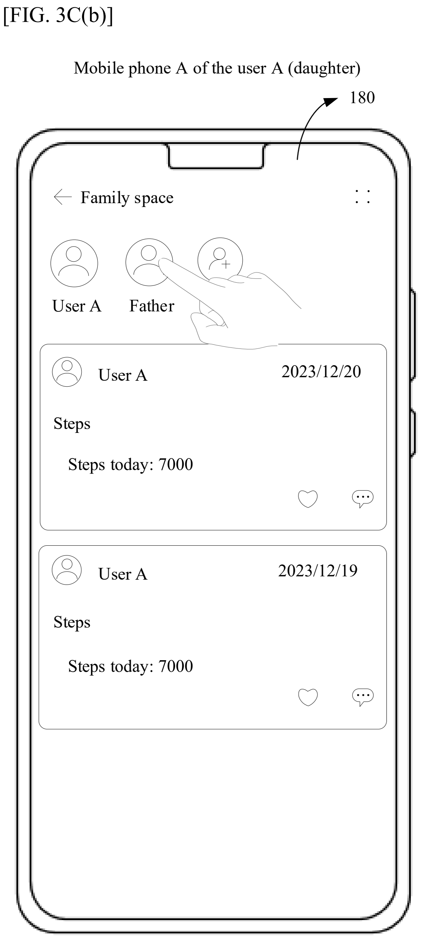

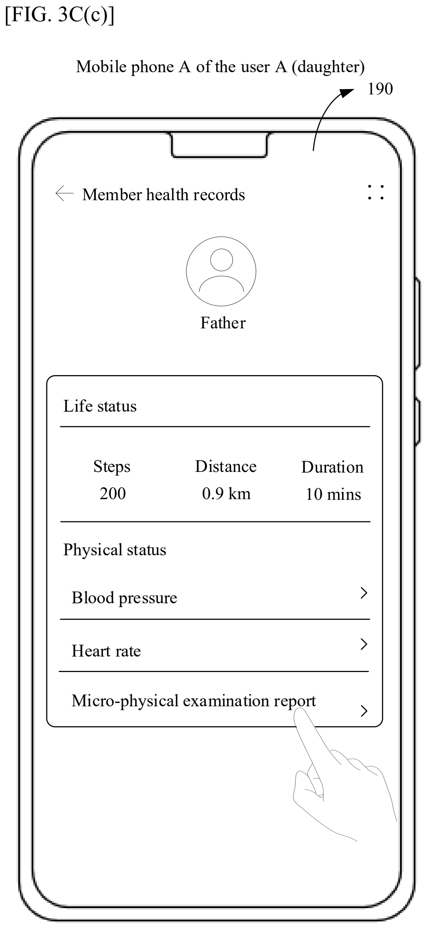

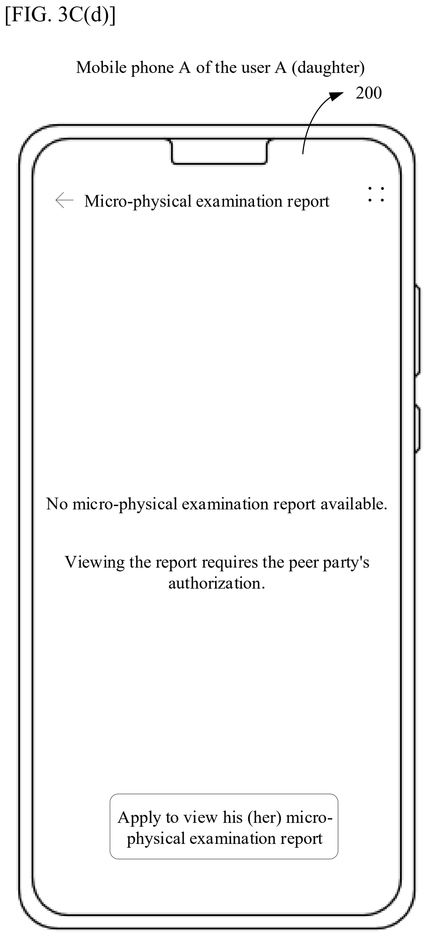

In some examples, when receiving the consent instruction sent by the cloud, the mobile phone A may output prompt information, to indicate that the user B accepts the friend request. For example, refer to FIG. 3C(a). The mobile phone A displays prompt information: Father has accepted your friend request. When receiving an operation on a "View" button, the mobile phone A may display an interface 180 shown in FIG. 3C(b). The interface 180 is an interface of the health and exercise application on the mobile phone A. The interface 180 includes a profile photo and/or a nickname of an added friend, for example, the father.

In some embodiments, after adding the user B (father) as a friend, the user A (daughter) may view the daily behavior and/or the physical status of the user B. It is assumed that the daughter wants to view the daily behavior of the father. Because the father sets the daily behavior to be disclosed to the daughter, the daughter can view the daily behavior of the father. It is assumed that the daughter wants to view the physical status of the father. Because the father sets the physical status not to be disclosed to the daughter, the daughter may apply to view the physical status of the father. Optionally, an implementation process includes S210 to S216 in the following.

S210: The mobile phone A sends an access request for data to the cloud, to request to access a piece of data of the account B, for example, physical status data.

S211: The cloud determines that the account A does not have access permission for the piece of data.

S212: The cloud sends, to the mobile phone A, prompt information indicating no permission to have a view.

For example, as shown in FIG. 3C(b), when receiving an operation on the profile photo and/or the nickname of the father, the mobile phone A displays an interface 190 shown in FIG. 3C(c). The interface 190 includes various pieces of data of the father. When receiving an operation on an option of a micro-physical examination report, the mobile phone A displays an interface 200 shown in FIG. 3C(d). The interface 200 displays prompt information: No micro-physical examination report is available. Viewing the micro-physical examination report requires the peer party's authorization. This is because in FIG. 3B(c), the father sets the micro-physical examination report not to be disclosed to the daughter.

S213: The mobile phone A sends an application to the cloud, to apply to view data. The application includes the account B.

For example, in FIG. 3C(d), the mobile phone A displays an "Apply to view his (her) micro-physical examination report" button. When receiving an operation on the button, the mobile phone A sends the application to the cloud.

S214: The cloud sends an application to the mobile phone B. The application includes the account A.

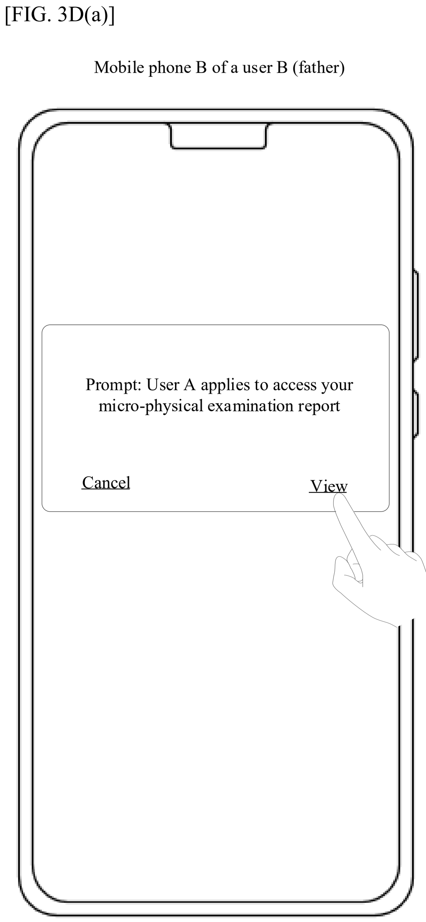

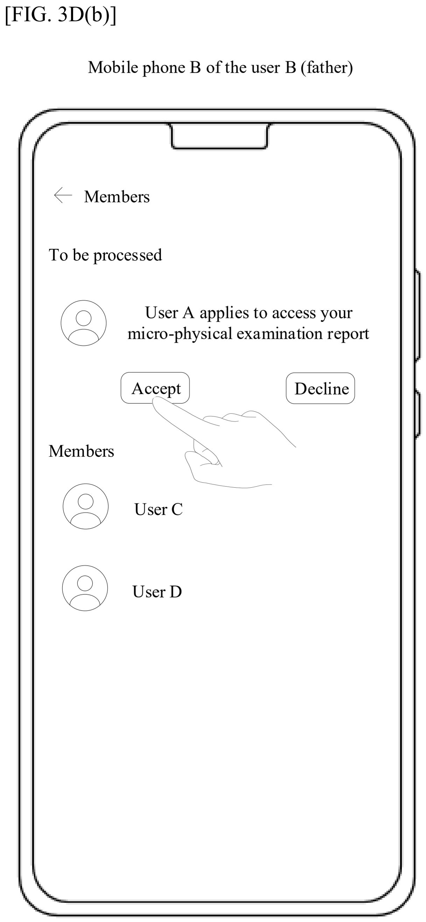

In some examples, when receiving the application, the mobile phone B may output prompt information, to indicate that the user A applies to view the data. For example, refer to FIG. 3D(a). The mobile phone B displays prompt information: User A applies to access your micro-physical examination report.

S215: The mobile phone B sends the consent instruction to the cloud, to indicate that the application of the user A is accepted.

FIG. 3D(a) is still used as an example. The mobile phone B displays a "View" button. When receiving an operation on the "View" button, the mobile phone B displays the interface shown in FIG. 3D(b), where the interface includes an "Accept" button and a "Decline" button. When receiving an operation on the "Accept" button, the mobile phone B sends the consent instruction to the cloud. It should be noted that, in FIG. 3D, the user B may disclose the micro-physical examination report to the user A permanently or temporarily. This is not limited in embodiments of this application.

S216: The cloud sends a consent instruction to the mobile phone A.

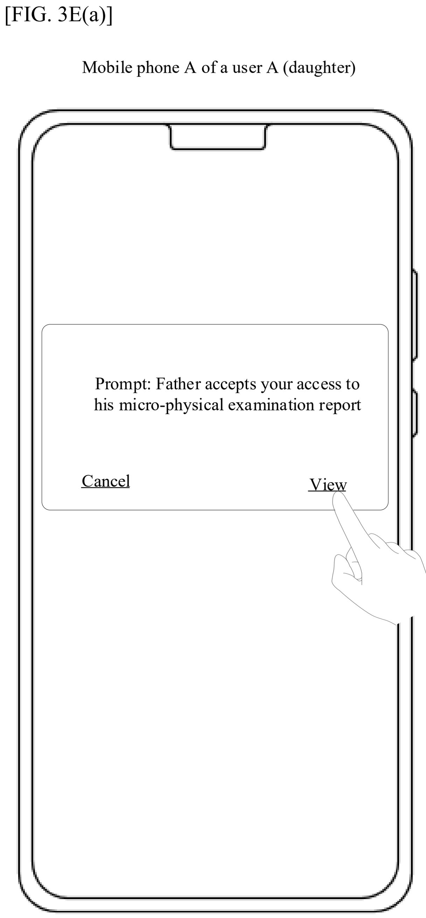

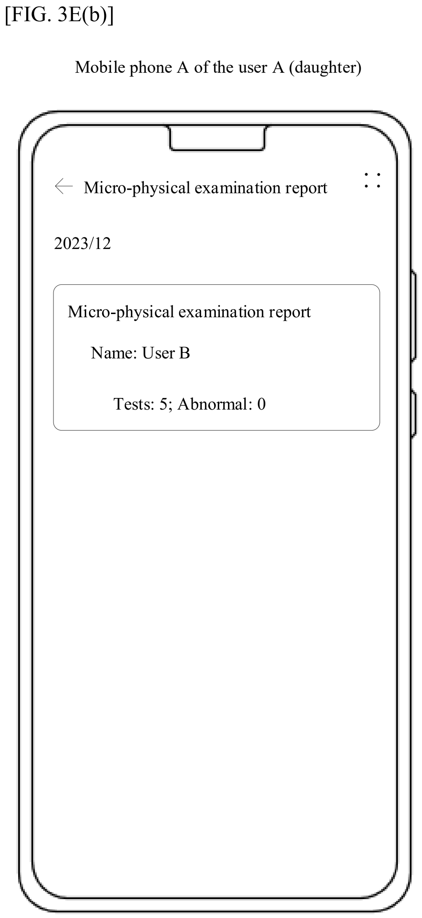

In some examples, after receiving the consent instruction, the mobile phone A may output prompt information, to indicate that the user B accepts the application. For example, refer to FIG. 3E(a). The mobile phone A displays prompt information: Father accepts your access to his micro-physical examination report.

S217: The mobile phone A sends the access request for the data to the cloud, to request to access a piece of data of the account B, for example, the physical status data.

S218: The cloud sends the data to the mobile phone A.

FIG. 3E(a) is still used as an example. The mobile phone A may display a "View" button. When receiving an operation on the "View" button, the mobile phone A sends the access request for the data to the cloud. After receiving data returned by the cloud, the mobile phone A displays an interface shown in FIG. 3E(b), where the interface includes the micro-physical examination report of the user B.

Optionally, S210 to S216 in FIG. 2 may be performed or not performed. Therefore, S210 to S216 in FIG. 2 are represented by using dashed lines. For example, S210 to S216 are not performed. For example, in S207, the consent instruction sent by the mobile phone B to the cloud includes the data access permission of the user A, to indicate disclosure of all the data to the user A. In this case, the user A does not need to apply for the data access permission. Therefore, S210 to S216 do not need to be performed.

In some examples, after disclosing some data to the user A, the user B may further cancel disclosure of the data to the user A. For example, in FIG. 3D, after the father discloses the micro-physical examination report to the daughter, the father may cancel disclosure of the micro-physical examination report to the daughter. A cancellation manner may include an automatic manner or a manual manner. The automatic manner is used as an example. For example, if the mobile phone B determines that duration for which a piece of data is disclosed to the user A reaches preset duration, the mobile phone B automatically cancels disclosure of the piece of data to the user A. The manual manner is used as an example. For example, the mobile phone B may display an interface shown in FIG. 3B(c), and cancel disclosure of some data to the user A on the interface. A manner in which the mobile phone B displays the interface in FIG. 3B(c) is not limited in embodiments of this application. Similarly, after disclosing a piece of data to the user B, the user A may also cancel disclosure of the piece of data to the user B based on the same principle.

In some embodiments, after the user B discloses some data to the user A, if the user A views the data, the mobile phone B may display prompt information, to indicate that the user A has viewed the data. For example, in FIG. 3D, the father discloses the micro-physical examination report to the daughter. If the daughter views the micro-physical examination report, the mobile phone B of the father may display prompt information that the daughter has viewed the micro-physical examination report. Optionally, the mobile phone B may further display view time.

In some other embodiments, after the user A adds the user B as a friend, the cloud may further notify the user A of a status of the user B, where the status of the user B includes the daily behavior and/or the physical status of the user B.

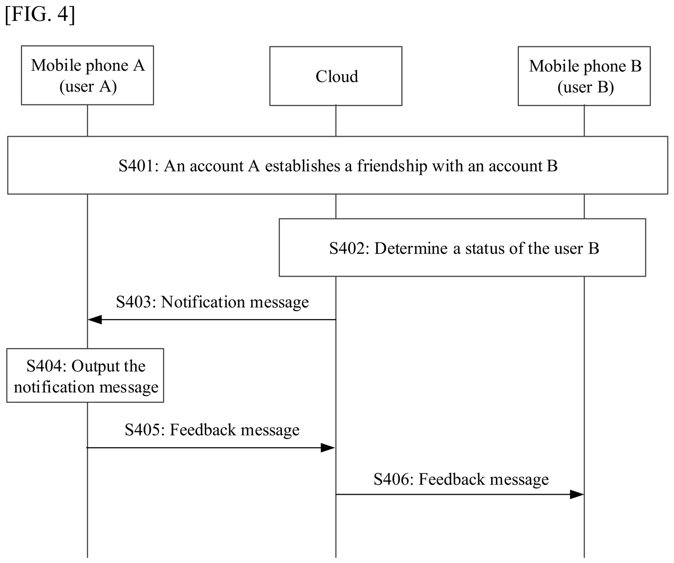

FIG. 4 is a schematic flowchart of a health management method according to an embodiment of this application. The method may be applied to the communication system shown in FIG. 1. As shown in FIG. 4, the method includes the following steps.

S401: An account A of a mobile phone A establishes a friendship with an account B of a mobile phone B.

Optionally, for an implementation principle of S401, refer to S201 to S209 in FIG. 2. Details are not described again.

S402: Determine a status of a user B.

In this embodiment of this application, the status of the user B (father) may include daily behavior and/or a physical status of the user B. Optionally, the status of the user B may include at least one of the following.
(1) The user B executes a first preset event. For example, the first preset event may include taking medicine, having a meal, drinking water, exercising, measuring blood pressure, measuring a heart rate, a physical examination, or the like. For example, the first preset event is the physical examination, and that the user B executes the first preset event may include: The user B uses a micro-physical examination function of a watch to perform the physical examination, and obtains a micro-physical examination report.
(2) The user B does not execute a second preset event. For example, the second preset event may include taking medicine, having a meal, exercising, a physical examination, or the like. Optionally, the second preset event may be the same as or different from the first preset event. For example, that the user B does not execute the second preset event may include that the user B does not execute the second preset event for more than specific duration. For example, the user B does not use, for more than first duration, a physical examination function of the watch to perform a physical examination. Alternatively, the user B does not exercise or has an amount of exercise less than a first threshold for more than second duration. For example, a quantity of steps of the user B is less than the first threshold for more than 4 hours. For example, the quantity of steps is 0.
(3) The user B triggers a specific function. For example, the user B triggers an SOS button of the watch, or the user B triggers a fall detection function of the watch and detects a fall.
(4) The user B moves out of a first geo-fence and/or moves into a second geo-fence. For example, the first geo-fence may include a home, a housing estate, or the like. For example, the second geo-fence includes a street, a seaside, or the like.
(5) A physical examination result of the user B is abnormal. For example, the micro-physical examination report of the user B is abnormal, a blood pressure measurement result is abnormal, a heart rate measurement result is abnormal, or the like.

Optionally, S402 may be performed by the mobile phone B locally, or may be performed by a cloud. This is not limited in embodiments of this application.

S403: The cloud sends a notification message to the mobile phone A, to notify the status of the user B.

S404: The mobile phone A outputs the notification message.

In some embodiments, the notification message may include one or more shortcuts, so that the user A can quickly view, contact, comment on, or remind the user B. In some other embodiments, the notification message may not include the shortcut. In this way, the mobile phone A may display one or more shortcuts based on the notification message. The following examples are separately used for description.

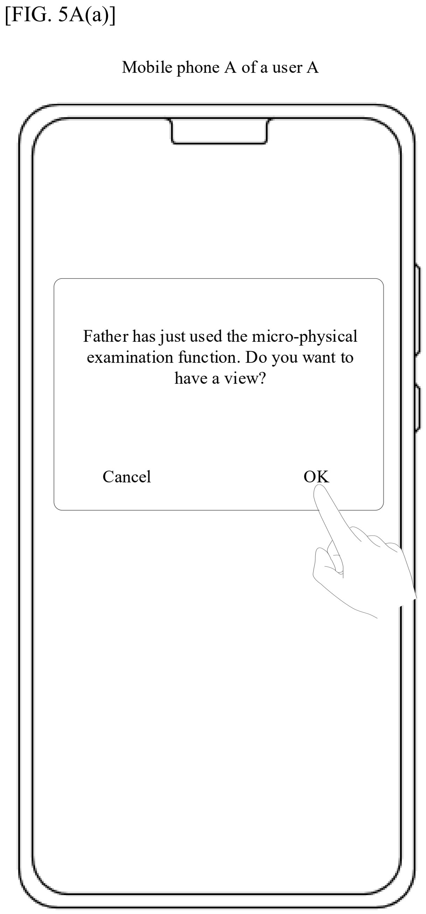

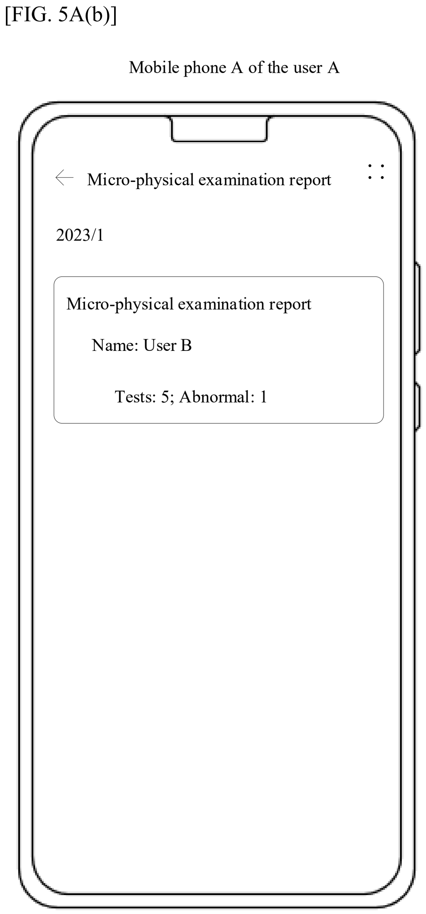

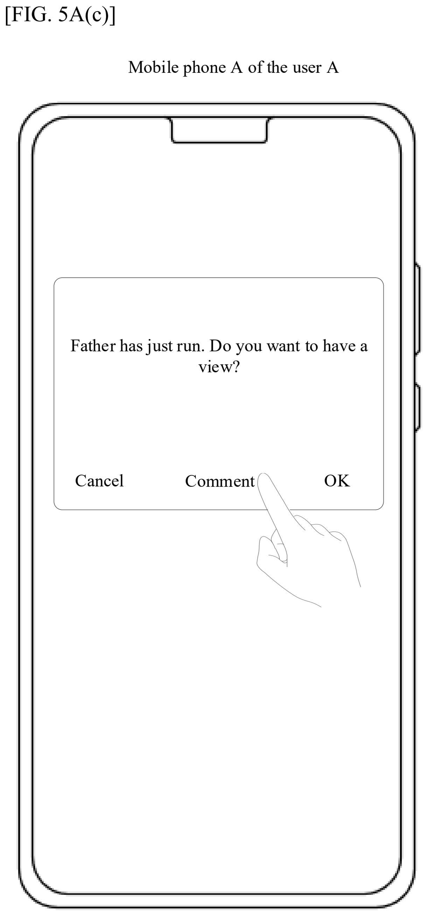

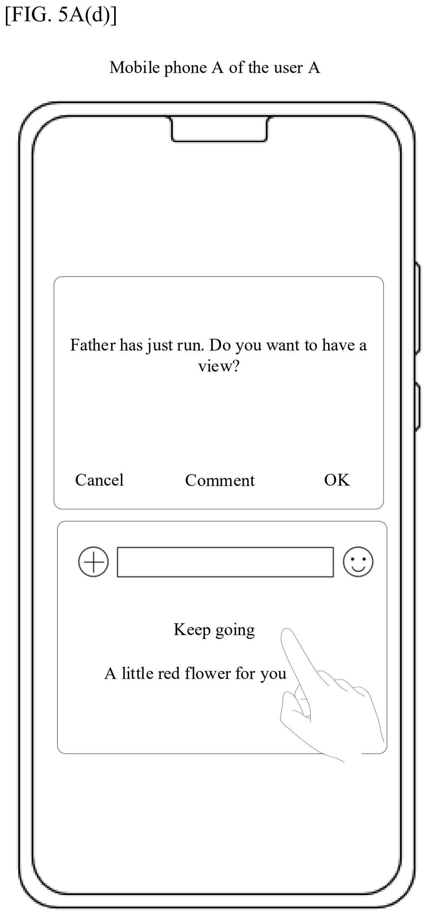

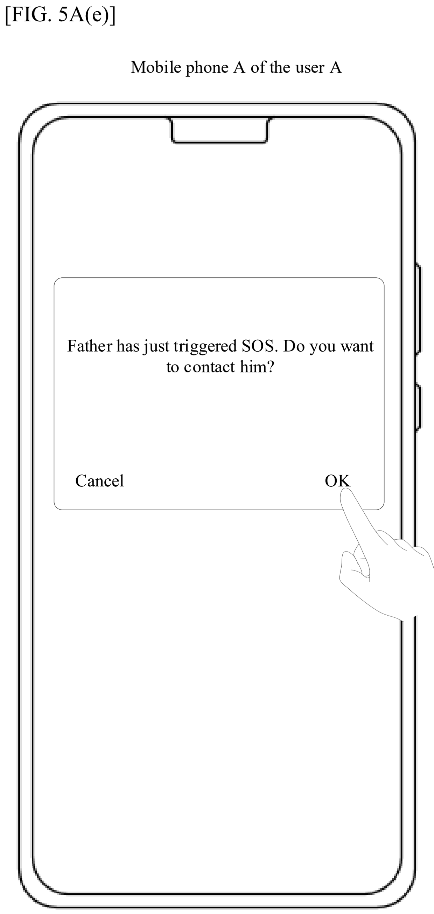

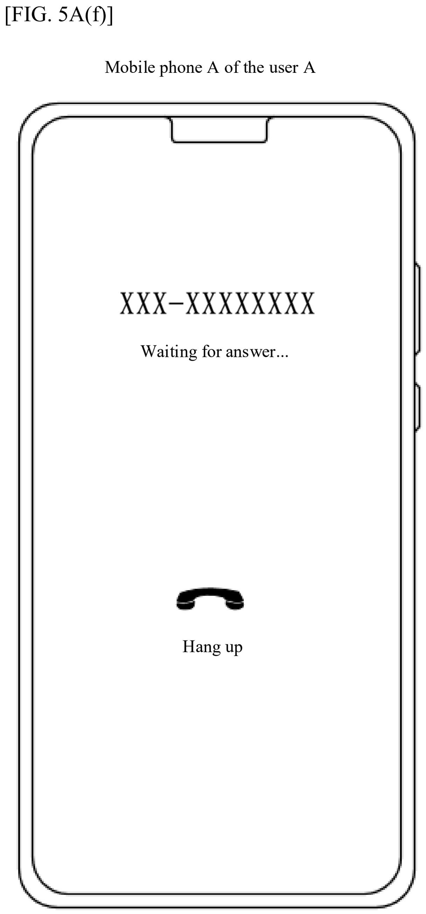

In some examples, after the user B executes an event, the user A may be notified, and the user A may view an execution result of the user B. For example, after the user B uses the micro-physical examination function, the user A may be notified, and the user A may view the micro-physical examination report of the user B. For example, as shown in FIG. 5A(a), the mobile phone A displays a notification message: Father has just used the micro-physical examination function. Do you want to have a view? The mobile phone A further displays an "OK" button and a "Cancel" button. When receiving an operation on the "OK" button, the mobile phone A displays the micro-physical examination report of the father, as shown in FIG. 5A(b).

In some other examples, after the user B executes an event, the user A may be notified, and the user A may comment on behavior of the user B. For example, after the user B exercises, the user A may be notified, and the user A may make a comment. For example, as shown in FIG. 5A(c), the mobile phone A displays a notification message: Father has just run. Do you want to have a view? The mobile phone A further displays an "OK" button, a "Cancel" button, and a "Comment" button. When the mobile phone A receives an operation on the "Comment" button, the father is commented, as shown in FIG. 5A(d).

In some other examples, after the user B triggers a specific event (for example, SOS or a fall), the user A may be notified, and the user A may quickly contact the user B. For example, after the user B triggers an SOS function, the user A may be notified. For example, as shown in FIG. 5A(e), the mobile phone A displays a notification message: Father has just triggered SOS. Do you want to contact him? The mobile phone A further displays an "OK" button and a "Cancel" button. When the mobile phone A receives an operation on the "OK" button, the father is contacted, as shown in FIG. 5A(f). Optionally, a contact manner may include making a call, making a video/voice call, or the like.

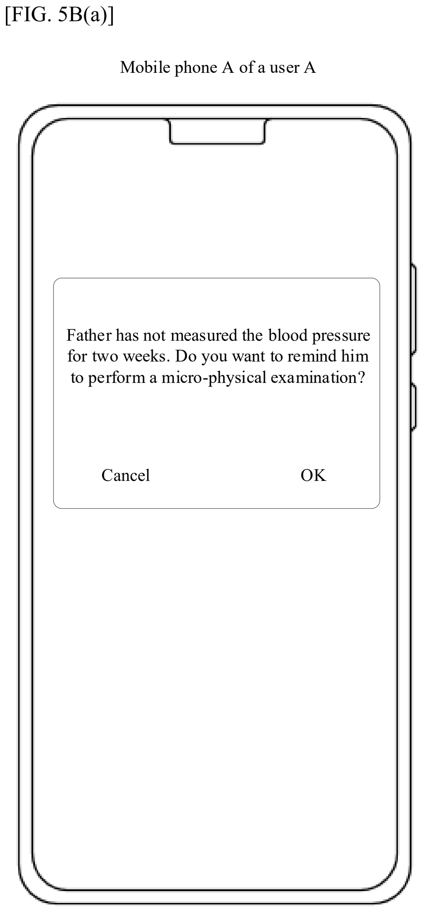

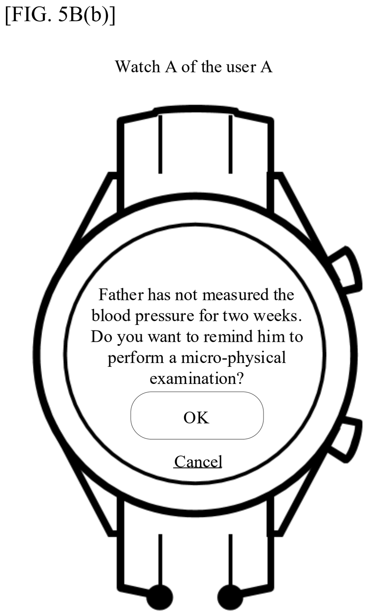

In some other examples, when the user B does not execute an event, the user A may be notified, and the user A may quickly contact the user B or send a reminder message to the user B, to remind the user B to execute the event. For example, when the user B does not use the micro-physical examination function for a long time, the user A may be notified, and the user A may remind the user B to use the micro-physical examination function to perform the micro-physical examination. For example, as shown in FIG. 5B(a), the mobile phone A displays a notification message: Father has not used the micro-physical examination function for two weeks. Do you want to remind him to perform a micro-physical examination? The mobile phone A further displays an "OK" button and a "Cancel" button. When receiving an operation on the "OK" button, the mobile phone A sends a reminder message to the cloud, to remind the father to perform the micro-physical examination.

It should be noted that in FIG. 5A(a) to FIG. 5A(f) and FIG. 5B(a), the cloud sends the notification message to the mobile phone A of the user A. In some other embodiments, the cloud may further send the notification message to another device of the user A. For example, if a watch A and the mobile phone A of the user A log in to the cloud by using the same account, the cloud further sends the notification message to the watch A. For example, as shown in FIG. 5B(b), the watch A displays the notification message: Father has not used the micro-physical examination function for two weeks. Do you want to remind him to perform a micro-physical examination? The watch A further displays an "OK" button and a "Cancel" button. When receiving an operation on the "OK" button, the watch A sends the reminder message to the cloud, to remind the father to perform the micro-physical examination.

S405: The mobile phone A sends a feedback message to the cloud. Optionally, the feedback message may be a comment message or a reminder message.

S406: The cloud sends the feedback message to the mobile phone B.

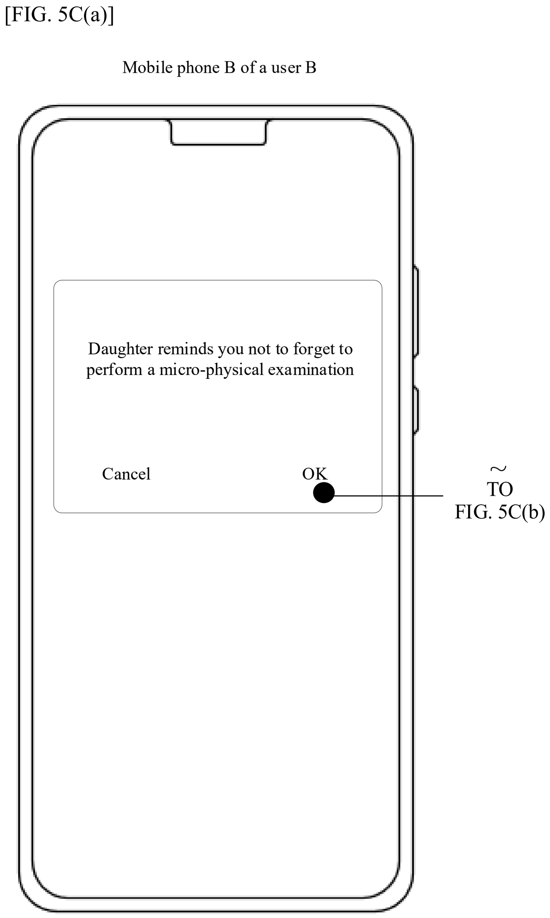

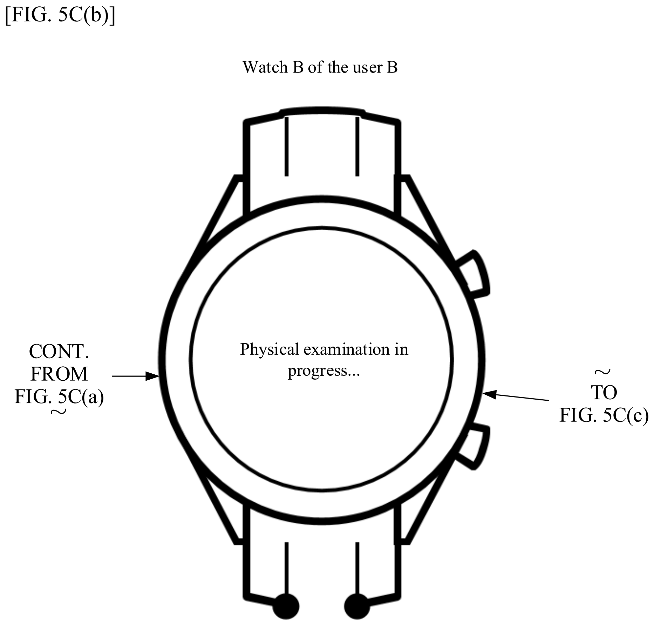

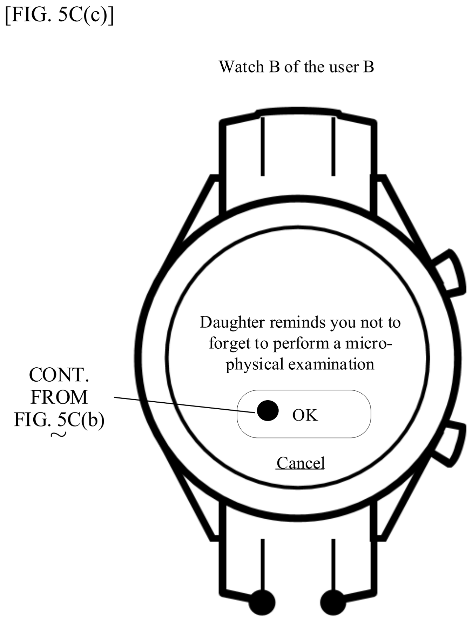

For example, the feedback message is a reminder message. As shown in FIG. 5C(a), the mobile phone B displays a reminder message: Daughter reminds you not to forget to perform a micro-physical examination. The mobile phone B further displays an "OK" button and a "Cancel" button. When the mobile phone B receives an operation on the "OK" button, a micro-physical examination function of a watch B is enabled to perform the physical examination, as shown in FIG. 5C(b). It should be noted that the cloud may further send the reminder message to another device of the father. For example, if the watch B and the mobile phone B of the father log in to the cloud by using the same account, the cloud further sends prompt information to the watch B. For example, as shown in FIG. 5C(c), the watch B displays prompt information: Daughter reminds you not to forget to perform a micro-physical examination. The watch B further displays an "OK" button and a "Cancel" button. When receiving an operation on the "OK" button, the watch B enables a micro-physical examination function to perform the physical examination, as shown in FIG. 5C(b).

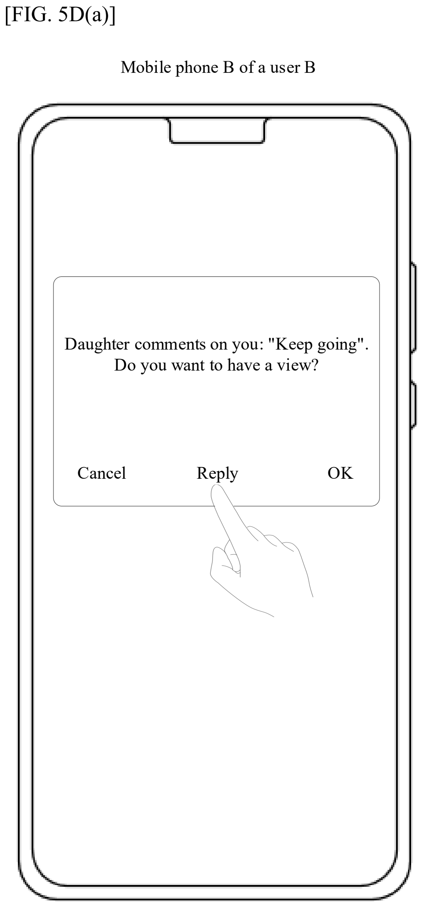

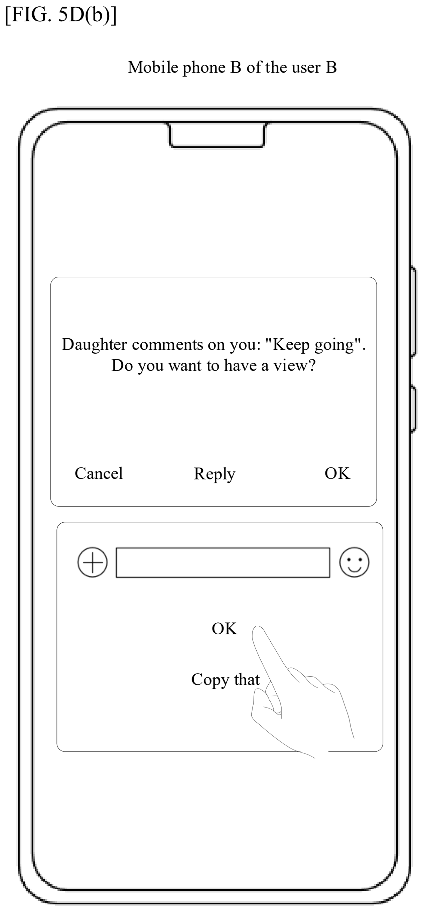

For example, the feedback message is a comment message. As shown in FIG. 5D(a), the mobile phone B displays a comment message: Daughter comments on you: "Keep going". Do you want to have a view? The mobile phone B further displays an "OK" button and a "Reply" button. When the mobile phone B receives an operation on the "OK" button, comment content is viewed. When the watch B receives an operation on the "Reply" button, reply may be quickly made to the comment, as shown in FIG. 5D(b). Optionally, after receiving a reply message of the mobile phone B, the mobile phone A of the daughter may also display an interface similar to FIG. 5D(a), for example, display: Father replies to your comment. Do you want to have a view? The mobile phone A may further display a "Reply" button and an "OK" button. The daughter may further reply quickly by using the "Reply" button.

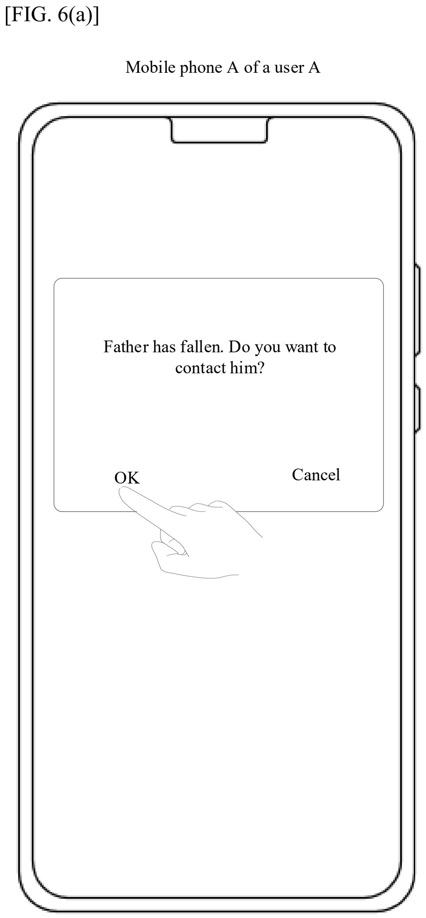

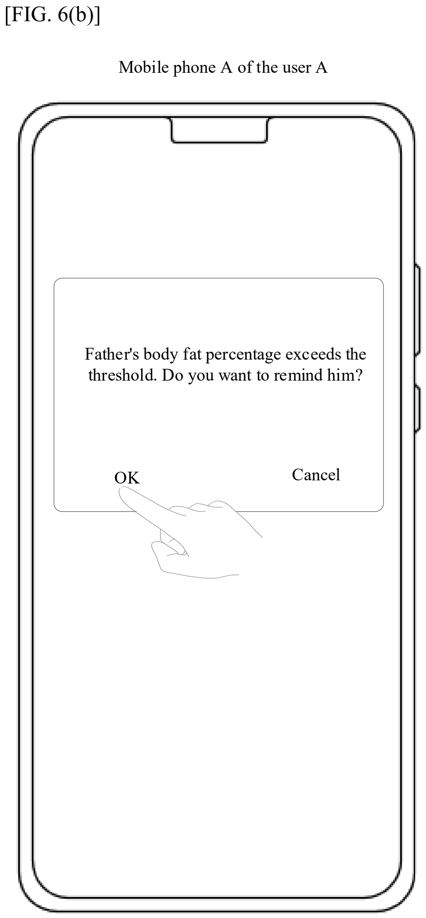

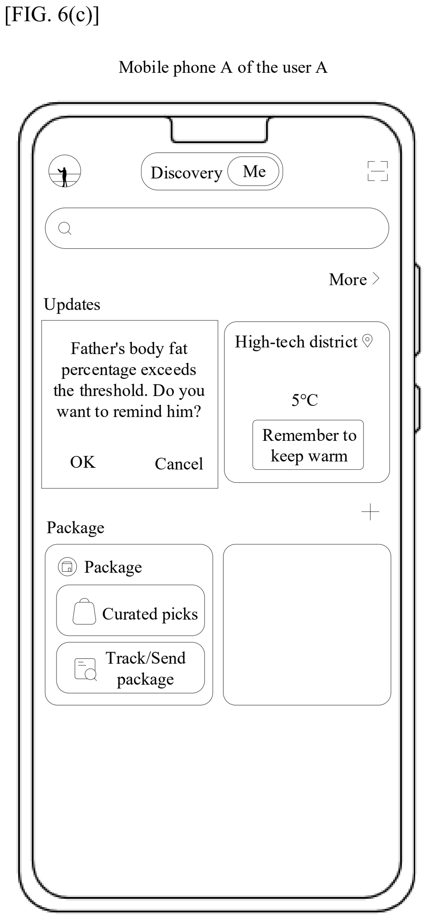

As described above, the status of the user B in S402 includes at least one of (1) to (5). In some examples, the foregoing (1) to (5) may be classified. For example, (1), (2), and (5) are classified as non-emergency situations, and (3) to (4) are classified as emergency situations. Optionally, a classification manner is not limited in embodiments of this application. In some examples, for the emergency situation, after the cloud sends the notification message to the mobile phone A of the daughter, the notification message on the mobile phone A may dominate a screen for display. For example, as shown in FIG. 6(a), the mobile phone A displays a notification message: Father has fallen. Do you want to contact him? The mobile phone A further displays an "OK" button and a "Cancel" button. In this case, if the user A taps the "Cancel" button, no response is made, or it is indicated, through vibration or in another manner, that display of the notification message cannot be canceled. If the user A taps the "OK" button, the user A sends a reminder message to the mobile phone B or contacts the user B (for example, makes a call). In this case, display of the notification message is canceled. Optionally, in this case, the mobile phone A may alternatively not display the "Cancel" button, but display only the "OK" button. In some other examples, in the non-emergency situation, after the cloud sends the notification message to the mobile phone A of the daughter, the notification message on the mobile phone A may not dominate the screen for display. For example, refer to FIG. 6(b). The mobile phone A displays a notification message: Father's body fat percentage exceeds the threshold. Do you want to contact him? The mobile phone A displays an "OK" button and a "Cancel" button. If the user A taps the "Cancel" button, display of the notification message is canceled. If the user A taps the "OK" button, the user A sends a reminder message to the mobile phone B or contacts the user B (for example, makes a call). Optionally, in this case, the notification message may be displayed at any location of the mobile phone A. For example, as shown in FIG. 6(c), the notification message may alternatively be displayed on a leftmost screen of the mobile phone A.

In the foregoing embodiment, for example, the cloud notifies the user A (daughter) of the status of the user B (father). It may be understood that the cloud may further notify another person, for example, a user C. For example, friends of the user B recorded in the cloud include the user A and the user C. In this case, after determining the status of the user B, the cloud may separately notify the user A and the user C. Alternatively, the cloud may determine a target user from the user A and the user C, and send the notification message to the target user. Optionally, the cloud determines the target user in a plurality of manners. For example, a user is randomly selected from the user A and the user C, or a user that is in the user A and the user C and that interacts with the user B for a larger quantity of times is determined as the target user, or the target user is determined based on a priority relationship between the user A and the user C. Optionally, the priority relationship may be set by the user B. For example, the user B is the father, the user A is the daughter, and the user C is a son. After determining the status of the father, the cloud may separately notify the daughter and the son, for example, separately send the notification message to the mobile phone of the daughter and a mobile phone of the son. Therefore, both the mobile phone of the daughter and the mobile phone of the son display the notification message. Optionally, assuming that the mobile phone of the daughter sends the reminder message to the mobile phone of the father, the mobile phone of the son may cancel display of the notification message. Optionally, the mobile phone of the son may further output prompt information, to indicate that the daughter has reminded the father. It is assumed that both the mobile phone of the daughter and the mobile phone of the son send reminder messages to the mobile phone of the father. In this case, the mobile phone of the father receives two reminder messages, and the reminder messages on the mobile phone of the father may also dominate the screen for display. For example, in FIG. 5C(a), on the mobile phone B of the father, the reminder message dominates the screen for display. When receiving an operation on the "Cancel" button, the mobile phone B makes no response, or indicates, through vibration or in another manner, that display of the reminder message cannot be canceled. When the mobile phone B receives an operation on the "OK" button or determines that the user B completes the micro-physical examination, the mobile phone B may cancel display of the reminder message.

In the foregoing embodiment, the user A (daughter) may view the status of the user B (father) by using the mobile phone A, including the daily behavior and/or the physical status of the user B. In some other embodiments, the user A may further help the user B with consultation based on the physical status data of the user B.

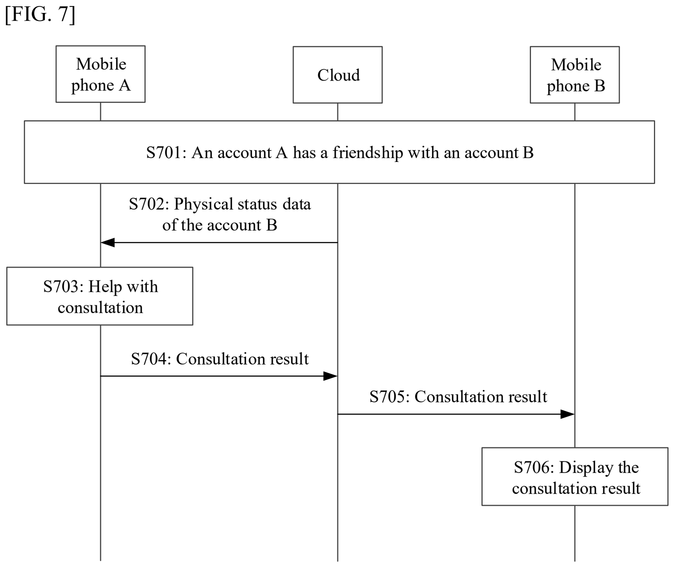

For example, FIG. 7 is a schematic flowchart of a health management method according to an embodiment of this application. The method may be applied to the communication system shown in FIG. 1. As shown in FIG. 7, the method includes the following steps.

S701: An account A of a mobile phone A establishes a friendship with an account B of a mobile phone B.

Optionally, for an implementation principle of S701, refer to S201 to S209 in FIG. 2. Details are not described herein again.

S702: View physical status data of the account B by using the mobile phone A. Optionally, for a process of viewing the physical status data of the user B by using the mobile phone A, refer to the foregoing description. Details are not described herein again.

S703: Help with consultation based on the physical status data of the account B by using the mobile phone A.

S704: The mobile phone A sends a consultation result to a cloud.

S705: The cloud sends the consultation result to the mobile phone B.

S706: The mobile phone B displays the consultation result.

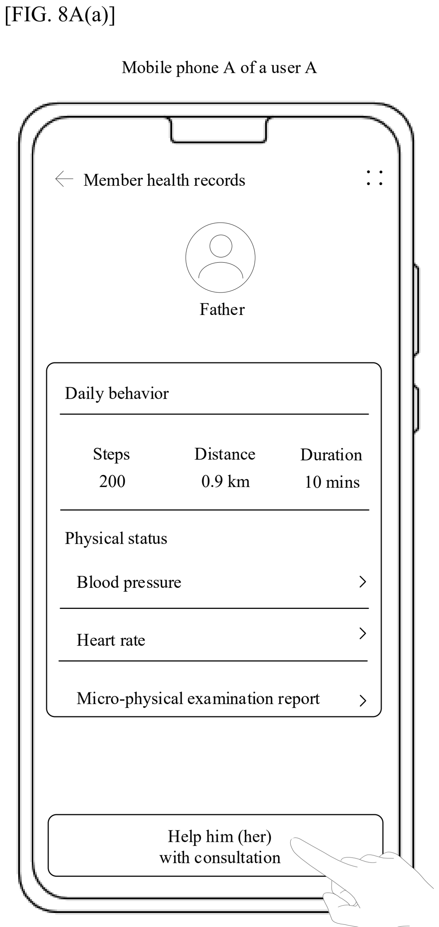

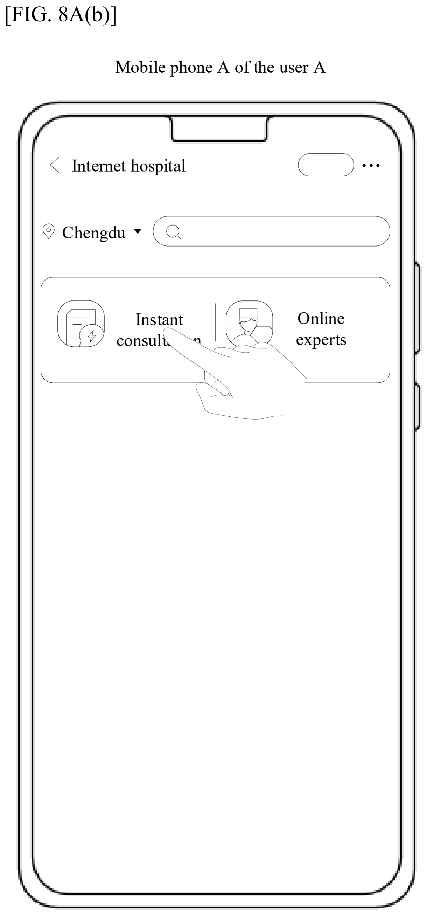

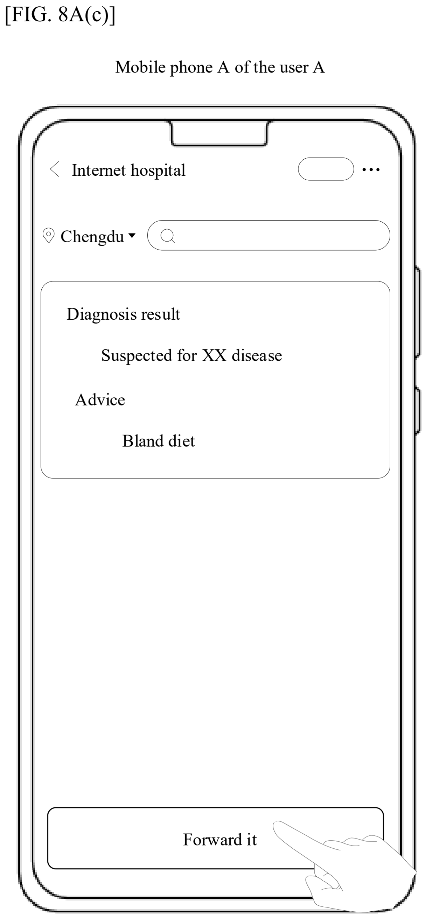

For example, refer to FIG. 8A(a). The mobile phone A displays an interface, where the interface includes the physical status data of the user B. The mobile phone A further displays a "Help him (her) with consultation" button. When receiving an operation on the "Help him (her) with consultation" button, the mobile phone A displays an interface shown in FIG. 8A(b), where the interface includes an online consultation button. When receiving an operation on the online consultation button, the mobile phone A sends the physical status data of the user B to a diagnosis platform (for example, a platform provided by a hospital). After obtaining a diagnosis result, the mobile phone A may display an interface shown in FIG. 8A(c), where the interface includes the diagnosis result. Optionally, after the user B is helped with online consultation by using the mobile phone A, the mobile phone A may further send the consultation result to the user B. For example, in FIG. 8A(c), when receiving a button for forwarding it, the mobile phone A sends the diagnosis result to the user B by using the cloud.

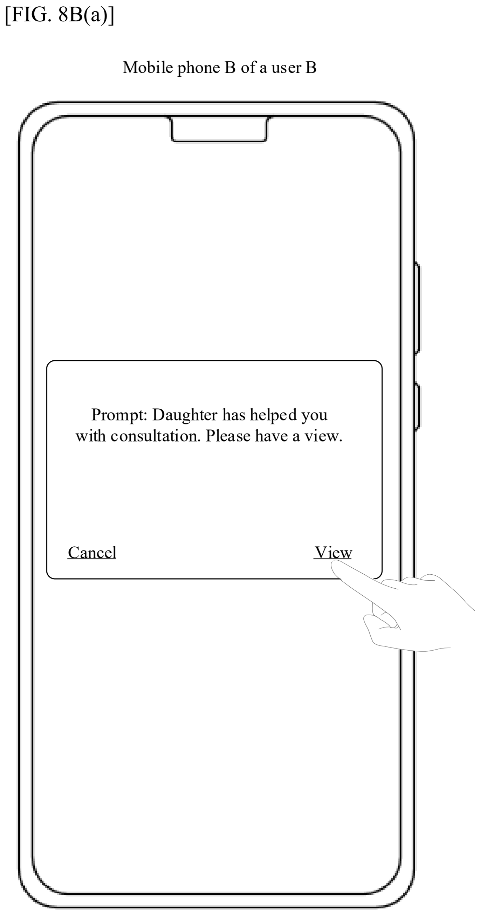

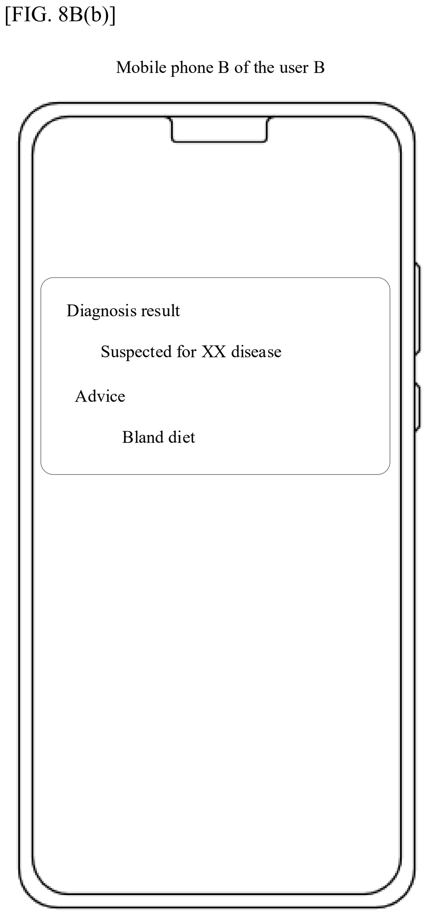

In some examples, when receiving the diagnosis result sent by the mobile phone A, the mobile phone B may output prompt information. For example, refer to FIG. 8B(a). The mobile phone B displays prompt information: Daughter has helped you with consultation. Please have a view. When receiving an operation on a "View" button, the mobile phone B displays an interface shown in FIG. 8B(b), where the interface includes the diagnosis result.

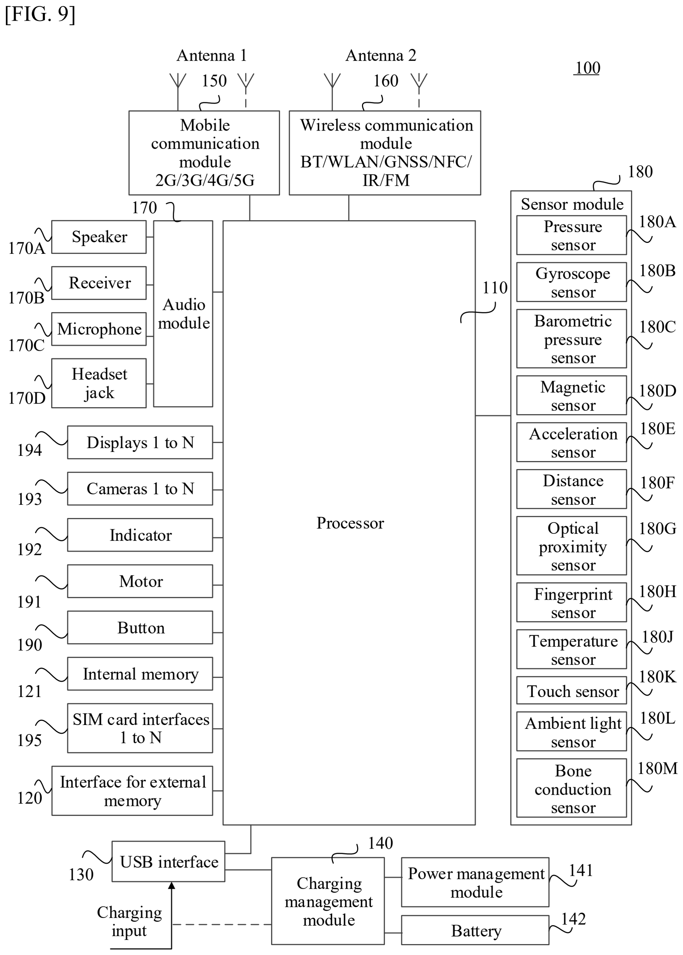

FIG. 9 is a diagram of a structure of an electronic device according to an embodiment of this application. The electronic device may be the mobile phone A or the mobile phone B described above. As shown in FIG. 9, the electronic device may include a processor 110, an interface 120 for external memory, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors. The controller may be a nerve center and a command center of the electronic device. The controller may generate an operation control signal based on instruction operation code and a time sequence signal, to complete control of instruction reading and instruction execution. A memory may be further disposed in the processor 110, and is configured to store instructions and data. In some embodiments, the memory in the processor 110 is a cache. The memory may store instructions or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may perform the shortcut display method provided in embodiments of this application. For example, the processor 110 may display a first interface by using the display 194, where the first interface includes N areas, and N is a positive integer. The N areas respectively correspond to N applications in the electronic device. A first area in the N areas includes at least one shortcut of a first application. The first area is any one of the N areas. The first application is an application that is in the N applications and that corresponds to the first area. The at least one shortcut is used to enable at least one function of the first application or display at least one interface of the first application. The shortcut in the first area and a shortcut in another area (an area, other than the first area, in the N areas) are independent of and irrelevant to each other.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be configured to: perform audio communication, and sample, quantize, and encode an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be configured to perform audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device 100. The processor 110 communicates with the display 194 through the DSI interface, to implement a display function of the electronic device 100.

The GPIO interface may be configured by using software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB interface, a micro USB interface, a USB type-C interface, or the like. The USB interface 130 may be configured to connect to a charger to charge the electronic device 100, or may be configured to transmit data between the electronic device 100 and a peripheral device, or may be configured to connect to a headset for playing audio through the headset. The interface may be further configured to connect to another electronic device such as an AR device.

It may be understood that an interface connection relationship between the modules that is shown in this embodiment of the present disclosure is merely an example for description, and does not constitute a limitation on the structure of the electronic device 100. In some other embodiments of this application, the electronic device 100 may alternatively use an interface connection manner different from that in the foregoing embodiment, or use a combination of a plurality of interface connection manners.

A wireless communication function of the electronic device may be implemented by using the antenna 1, the antenna 2, the mobile communication module 150, the wireless communication module 160, the modem processor, the baseband processor, and the like. The antenna 1 and the antenna 2 are configured to transmit and receive an electromagnetic wave signal. Each antenna of the electronic device may be configured to cover one or more communication frequency bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 150 may provide a solution that is applied to the electronic device and that includes wireless communication such as 2G/3G/4G/5G. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, the at least some functional modules of the mobile communication module 150 may be disposed in a same component as at least some modules of the processor 110.

The wireless communication module 160 may provide a solution to wireless communication that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processor module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device, the antenna 1 and the mobile communication module 150 are coupled, and the antenna 2 and the wireless communication module 160 are coupled, so that the electronic device can communicate with a network and another device by using a wireless communication technology.

The display 194 is configured to display a display interface of an application, and the like. The display 194 includes a display panel. In some embodiments, the electronic device may include one or N displays 194, where N is a positive integer greater than 1.

The electronic device 100 may implement a photographing function through the ISP, the camera 193, the video codec, the GPU, the display 194, the application processor, and the like. The ISP is configured to process data fed back by the camera 193.

The internal memory 121 may be configured to store computer-executable program code. The executable program code includes instructions. The processor 110 runs the instructions stored in the internal memory 121, to perform various function applications and data processing of the electronic device. The internal memory 121 may include a program storage area and a data storage area. The program storage area may store an operating system, software code of at least one application, and the like. The data storage area may store data (for example, an image or a video) generated in a process of using the electronic device, and the like. In addition, the internal memory 121 may include a high-speed random access memory, or may include a non-volatile memory, for example, at least one magnetic disk storage device, a flash storage device, or a universal flash storage.

The interface 120 for external memory may be configured to connect to an external memory card, such as a micro SD card, to extend a storage capability of the electronic device. The external memory card communicates with the processor 110 through the interface 120 for external memory, to implement a data storage function. For example, files such as pictures or videos are stored in the external memory card.

The electronic device may implement an audio function, for example, music playing or recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may listen to music by using one or more loudspeakers 170A, or may be used in a speaker scenario, for example, answering a hands-free call.

The receiver 170B, also referred to as an "earpiece", is configured to convert an audio electrical signal into a sound signal, and there may be one or more receivers 170B. When a call is answered or speech information is received through the electronic device 100, the receiver 170B may be put close to a human ear to listen to a voice.

The microphone 170C, also referred to as a "mike" or a "mic", is configured to convert a sound signal into an electrical signal.

The headset jack 170D is configured to connect to a wired headset.

The pressure sensor 180A is configured to sense a pressure signal, and can convert the pressure signal into an electrical signal. In some embodiments, the pressure sensor 180A may be disposed on the display 194.

The gyroscope sensor 180B may be configured to determine a motion posture of the electronic device. In some embodiments, angular velocities of the electronic device around three axes (that is, an x-axis, a y-axis, and a z-axis) may be determined by using the gyroscope sensor 180B. The gyroscope sensor 180B may be configured to implement image stabilization during photographing.

The barometric pressure sensor 180C is configured to measure barometric pressure. In some embodiments, the electronic device calculates an altitude based on a barometric pressure value measured by the barometric pressure sensor 180C, to assist in positioning and navigation.

The magnetic sensor 180D includes a Hall sensor. The electronic device may detect opening and closing of a flip cover by using the magnetic sensor 180D.

The acceleration sensor 180E may detect accelerations in various directions (usually on the three axes) of the electronic device. When the electronic device is static, a magnitude and a direction of gravity may be detected.

The distance sensor 180F is configured to measure a distance. The electronic device may measure the distance in an infrared manner or a laser manner.

The optical proximity sensor 180G may include, for example, a light-emitting diode (LED) and an optical detector, for example, a photodiode. The light-emitting diode may be an infrared light-emitting diode. The electronic device emits infrared light outwards through the light-emitting diode. The electronic device uses the photodiode to detect infrared reflected light from a nearby object. When detecting sufficient reflected light, the electronic device may determine that there is an object near the electronic device. When detecting insufficient reflected light, the electronic device may determine that there is no object near the electronic device.

The ambient light sensor 180L is configured to sense ambient light brightness. The electronic device may adaptively adjust brightness of the display 194 based on the sensed ambient light brightness.

The fingerprint sensor 180H is configured to collect a fingerprint.

The temperature sensor 180J is configured to detect a temperature.

The touch sensor 180K is also referred to as a "touch panel". The touch sensor 180K may be disposed in the display 194, and the touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of a touch event.

The bone conduction sensor 180M may obtain a vibration signal. In some embodiments, the bone conduction sensor 180M may obtain a vibration signal of a vibration bone of a human vocal-cord part.

The button 190 includes a power button, a volume button, and the like. The button 190 may be a mechanical button, or may be a touch button. The electronic device may receive button input, and generate button signal input related to user settings and function control of the electronic device. The motor 191 may generate a vibration prompt. The motor 191 may be configured to provide an incoming call vibration prompt and a touch vibration feedback. The indicator 192 may be an indicator, and may be configured to indicate a charging status and a power change, or may be configured to indicate a message, a missed call, a notification, and the like. The SIM card interface 195 is configured to connect to a SIM card. The SIM card may be inserted into the SIM card interface 195 or pulled out of the SIM card interface 195, to implement contact with or separation from the electronic device.

It may be understood that the components shown in FIG. 9 do not constitute a specific limitation on the electronic device. The electronic device in embodiments of the present invention may include more or fewer components than those in FIG. 9. In addition, a combination/connection relationship between the components in FIG. 9 may alternatively be adjusted and modified.

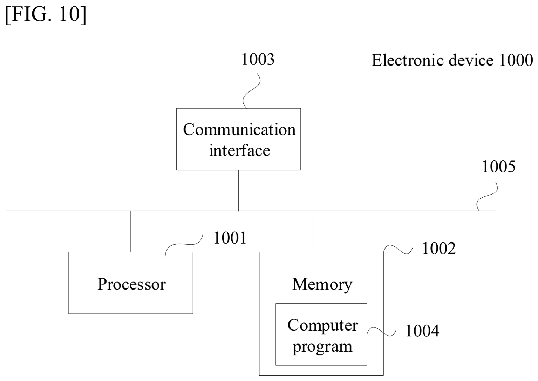

FIG. 10 is a diagram of a structure of an electronic device 1000 according to an embodiment of this application. The electronic device 1000 may be the first device (for example, the mobile phone A), the second device (for example, the mobile phone B), or the cloud described above. As shown in FIG. 10, the electronic device 1000 may include one or more processors 1001, one or more memories 1002, a communication interface 1003, and one or more computer programs 1004. The foregoing components may be connected through one or more communication buses 1005. The one or more computer programs 1004 are stored in the memory 1002 and are configured to be executed by the one or more processors 1001, and the one or more computer programs 1004 include instructions. For example, when the electronic device 1000 is the first device (for example, the mobile phone A) described above, the instructions may be used to perform related steps of the first device (for example, the mobile phone A) in the foregoing corresponding embodiment, for example, perform related steps of the first device (for example, the mobile phone A) in FIG. 2, FIG. 4, or FIG. 7. For another example, when the electronic device 1000 is the second device (for example, the mobile phone B) described above, the instructions may be used to perform related steps of the second device (for example, the mobile phone B) in the foregoing corresponding embodiment, for example, perform related steps of the second device (for example, the mobile phone B) in FIG. 2, FIG. 4, or FIG. 7. For another example, when the electronic device 1000 is the cloud described above, the instructions may be used to perform related steps of the cloud in the foregoing corresponding embodiment, for example, perform related steps of the cloud in FIG. 2, FIG. 4, or FIG. 7. The communication interface 1003 is configured to implement communication between the electronic device 1000 and another device. For example, the communication interface may be a transceiver.

In the foregoing embodiments provided in this application, the method provided in embodiments of this application is described from the perspective in which the electronic device (for example, the mobile phone) is used as an execution body. To implement functions in the foregoing method provided in embodiments of this application, the electronic device may include a hardware structure and/or a software module, and the foregoing functions are implemented in a form of a hardware structure, a software module, or a combination of a hardware structure and a software module. Whether a function in the foregoing functions is performed by using the hardware structure, the software module, or the combination of the hardware structure and the software module depends on particular applications and design constraints of the technical solutions.

All or some of the foregoing embodiments may be implemented by using software, hardware, firmware, or any combination thereof. When software is used to implement the embodiments, all or some of the embodiments may be implemented in a form of computer program product. The computer program product includes one or more computer instructions. When the computer program instructions are loaded and executed on a computer, the procedure or functions according to embodiments of the present invention are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, DVD), a semiconductor medium (for example, a solid-state disk (Solid-State Disk, SSD)), or the like. In a case in which no conflict occurs, the solutions in the foregoing embodiments may be combined for use.

A person skilled in the art should understand that embodiments of this application may be provided as a method, a system, or a computer program product. Therefore, this application may use a form of hardware-only embodiments, software-only embodiments, or embodiments with a combination of software and hardware. In addition, this application may use a form of a computer program product that is implemented on one or more computer-usable storage media (including but not limited to a disk memory, a CD-ROM, an optical memory, and the like) that include computer-usable program code.

This application is described with reference to the flowcharts and/or block diagrams of the method, the device (system), and the computer program product according to this application. It should be understood that computer program instructions may be used to implement each process and/or each block in the flowcharts and/or the block diagrams and a combination of a process and/or a block in the flowcharts and/or the block diagrams. These computer program instructions may be provided for a general-purpose computer, a dedicated computer, an embedded processor, or a processor of another programmable data processing device to generate a machine, so that the instructions executed by the computer or the processor of the another programmable data processing device generate an apparatus for implementing a specific function in one or more processes in the flowcharts and/or in one or more blocks in the block diagrams.

These computer program instructions may alternatively be stored in a computer-readable memory that can indicate the computer or the another programmable data processing device to work in a specific manner, so that the instructions stored in the computer-readable memory generate an artifact that includes an instruction apparatus. The instruction apparatus implements a specific function in one or more processes in the flowcharts and/or in one or more blocks in the block diagrams.

These computer program instructions may alternatively be loaded onto the computer or the another programmable data processing device, so that a series of operations and steps are performed on the computer or the another programmable device, to generate computer-implemented processing. Therefore, the instructions executed on the computer or the another programmable device provide steps for implementing a specific function in one or more processes in the flowcharts and/or in one or more blocks in the block diagrams.

It is clearly that a person skilled in the art can make various modifications and variations to this application without departing from the scope of this application. This application is intended to cover these modifications and variations of this application provided that they fall within the scope of the claims of this application and an equivalent technology thereof.

## Claims

1. A health management method, applied to a first device, wherein the method comprises:
receiving a notification message, wherein the notification message is used to notify a first user of a status of a second user, the first user is a user corresponding to the first device, the second user has an association relationship with the first user, and the status of the second user comprises daily behavior and/or a physical status of the second user; and
displaying the notification message.

2. The method according to claim 1, wherein the status of the second user comprises at least one of the following:
the second user executes a first preset event;
the second user does not execute a second preset event; and
the second user triggers a specific function of a second device, wherein the second device is a device of the second user.

3. The method according to claim 2, wherein that the second user executes the first preset event comprises:
the second user uses a physical examination function of the second device to perform a physical examination, wherein the second device is a device corresponding to the second user.

4. The method according to claim 2, wherein that the second user does not execute the second preset event comprises:
the second user does not use, for more than first duration, a physical examination function of the second device to perform a physical examination, wherein the second device is a device corresponding to the second user; or
the second user does not exercise or has an amount of exercise less than a first threshold for more than second duration.

5. The method according to claim 2, wherein that the second user triggers the specific function of the second device comprises:
the second user triggers an SOS button of the second device; or
the second user triggers a fall detection function of the second device and it is detected that the second user falls.

6. The method according to claim 1, wherein the status of the second user comprises at least one of the following:
the second user moves out of a first geo-fence and/or moves into a second geo-fence; and
a physical examination result of the second user is abnormal.

7. The method according to any one of claims 1 to 6, wherein the notification message comprises a first shortcut, and the method further comprises:
receiving an operation on the first shortcut, and displaying the status of the second user.

8. The method according to any one of claims 1 to 7, wherein the notification message comprises a second shortcut, and the method further comprises:
receiving an operation on the second shortcut, and sending a first message to the second device, wherein the second device is a device of the second user, and the first message is used to comment on the status of the second user.

9. The method according to any one of claims 2 to 8, wherein the status of the second user comprises that the second user does not execute the second preset event, the notification message comprises a third shortcut, and the method further comprises:
receiving an operation on the third shortcut, and contacting the second user or sending a second message to the second device, wherein the second device is a device of the second user, and the second message is used to prompt the second user to execute the second preset event.

10. The method according to any one of claims 1 to 9, wherein the method further comprises:
when an operation used to cancel display of the notification message is received, making no response or outputting prompt information, wherein the prompt information indicates that display of the notification message is unable to be canceled; or
when an operation used to contact the second user is received, canceling display of the notification message.

11. The method according to any one of claims 1 to 10, wherein before receiving the notification message, the method further comprises:
sending a first request to the second device, wherein the first request is used to request to establish the association relationship between the first user and the second user, and the second device is a device of the second user; and
receiving a consent instruction of the second user.

12. The method according to any one of claims 1 to 11, wherein the method further comprises:
sending a second request to the second device, wherein the second request is used to request to obtain access permission for the status of the second user, and the second device is a device of the second user; and
receiving a consent instruction of the second user.

13. The method according to any one of claims 1 to 12, wherein the status of the second user comprises the physical status of the second user, and the method further comprises:
obtaining a diagnosis result based on the physical status of the second user; and
sending the diagnosis result to the second device, wherein the second device is a device of the second user.

14. A health management method, applied to a second device, wherein the method comprises:
determining a status of a second user, wherein the second user is a user corresponding to the second device, and the status of the second user comprises daily behavior and/or a physical status of the second user; and
sending a notification message to a first device, wherein the notification message is used to notify a first user of the status of the second user, the first user has an association relationship with the second user, and the first device is a device of the first user.

15. The method according to claim 14, wherein the status of the second user comprises at least one of the following:
the second user executes a first preset event;
the second user does not execute a second preset event; and
the second user triggers a specific function of a third device, wherein the third device is a device of the second user.

16. The method according to claim 15, wherein that the second user executes the first preset event comprises:
the second user uses a physical examination function of the third device to perform a physical examination, wherein the third device is a device corresponding to the second user.

17. The method according to claim 15, wherein that the second user does not execute the second preset event comprises:
the second user does not use, for more than first duration, a physical examination function of the third device to perform a physical examination, wherein the third device is a device corresponding to the second user; or
the second user does not exercise or has an amount of exercise less than a first threshold for more than second duration.

18. The method according to claim 15, wherein that the second user triggers the specific function of the third device comprises:
the second user triggers an SOS button of the third device; or
the second user triggers a fall detection function of the third device and it is detected that the second user falls.

19. The method according to claim 14, wherein the status of the second user comprises at least one of the following:
the second user moves out of a first geo-fence and/or moves into a second geo-fence; and
a physical examination result of the second user is abnormal.

20. The method according to any one of claims 14 to 19, wherein the method further comprises:
receiving a first message sent by the first device, wherein the first message is used to comment on the status of the second user.

21. The method according to claim 20, wherein the method further comprises:
displaying a first shortcut based on the first message; and
receiving an operation on the first shortcut, and viewing or replying to a comment on the second user.

22. The method according to any one of claims 15 to 19, wherein the status of the second user comprises that the second user does not execute the second preset event, and the method further comprises:
receiving a second message sent by the first device, wherein the second message is used to prompt the second user to execute the second preset event.

23. The method according to claim 22, wherein the method further comprises:
displaying a second shortcut based on the second message; and
receiving an operation on the second shortcut, and executing the second preset event.

24. The method according to claim 22 or 23, wherein the method further comprises:
displaying the second message; and
when an operation used to cancel display of the second message is received, making no response or outputting prompt information, wherein the prompt information indicates that display of the second message is unable to be canceled; or
after it is determined that the second user executes the second preset event, canceling display of the second message.

25. The method according to any one of claims 14 to 24, wherein the method further comprises:
displaying first prompt information, wherein the first prompt information indicates that the first user has viewed the status of the second user.

26. The method according to any one of claims 14 to 25, wherein before sending the notification message to the first device, the method further comprises:
receiving a first request sent by the first device, wherein the first request is used to request to establish the association relationship between the first user and the second user; and
receiving a first operation, wherein the first operation indicates consent to establishing the association relationship between the first user and the second user.

27. The method according to any one of claims 14 to 26, wherein the method further comprises:
receiving a second request sent by the first device, wherein the second request is used to request to obtain access permission for the status of the second user; and
receiving a second operation, wherein the first operation indicates consent to granting the access permission.

28. The method according to any one of claims 14 to 27, wherein the status of the second user comprises the physical status of the second user, and the method further comprises:
receiving a diagnosis result sent by the first device, wherein the diagnosis result is obtained based on the physical status of the second user; and
displaying the diagnosis result.

29. An electronic device, comprising:
a processor, a memory, and one or more programs, wherein
the one or more programs are stored in the memory, the one or more programs comprise instructions, and when the instructions are executed by the processor, the electronic device is enabled to perform the method steps according to any one of claims 1 to 28.

30. A communication system, comprising a first device and a second device, wherein the first device is configured to perform the method steps according to any one of claims 1 to 13; and
the second device is configured to perform the method steps according to any one of claims 14 to 28.

31. A computer-readable storage medium, wherein the computer-readable storage medium is configured to store a computer program, and when the computer program is run on a computer, the computer is enabled to perform the method according to any one of claims 1 to 28.
